(19) Europäisches Patentamt
European Patent Office
Office européen des brevets

(11) EP 3 591 040 A1

(12) **EUROPEAN PATENT APPLICATION**
published in accordance with Art. 153(4) EPC

(43) Date of publication:
**08.01.2020 Bulletin 2020/02**

(21) Application number: **18761238.7**

(22) Date of filing: **02.03.2018**

(51) Int Cl.:
***C12N 5/10*** *(2006.01)* ***A61K 35/545*** *(2015.01)*
***A61P 3/10*** *(2006.01)* ***C12N 5/071*** *(2010.01)*

(86) International application number:
**PCT/JP2018/007965**

(87) International publication number:
**WO 2018/159805 (07.09.2018 Gazette 2018/36)**

(84) Designated Contracting States:
**AL AT BE BG CH CY CZ DE DK EE ES FI FR GB GR HR HU IE IS IT LI LT LU LV MC MK MT NL NO PL PT RO RS SE SI SK SM TR**
Designated Extension States:
**BA ME**
Designated Validation States:
**KH MA MD TN**

(30) Priority: **03.03.2017 JP 2017040219**

(71) Applicants:
- **Kyoto University**
  **Kyoto-shi, Kyoto 606-8501 (JP)**
- **Takeda Pharmaceutical Company Limited**
  **Osaka-shi, Osaka 541-0045 (JP)**

(72) Inventors:
- **OSAFUNE, Kenji**
  **Kyoto-shi**
  **Kyoto 606-8501 (JP)**
- **TOYODA, Taro**
  **Kyoto-shi**
  **Kyoto 6068501 (JP)**
- **TAKEKAWA, Shiro**
  **Fujisawa-shi**
  **Kanagawa 251-0012 (JP)**
- **NAKAMURA, Goshi**
  **Tokyo 141-6017 (JP)**
- **ITO, Ryo**
  **Fujisawa-shi**
  **Kanagawa 251-0012 (JP)**

(74) Representative: **Wallin, Nicholas James**
**Withers & Rogers LLP**
**4 More London Riverside**
**London SE1 2AU (GB)**

# (54) PANCREATIC PROGENITOR CELL PRODUCTION METHOD

(57) The present invention relates to a method for producing pancreatic progenitor cells from pluripotent stem cells. More specifically, the present invention relates to a method for producing pancreatic progenitor cells, comprising causing the action of a factor having the inhibitory activity for cyclin-dependent kinase 8 and/or cyclin-dependent kinase 19 (hereinafter, also abbreviated to CDK8/19).

EP 3 591 040 A1

**Description**

Technical Field

[Related Application]

The present specification encompasses the contents described in the specification of Japanese Patent Application No. 2017-040219 (filed on March 3, 2017) on which the priority of the present application is based.

[Technical Field]

The present invention relates to a method for producing pancreatic progenitor cells from pluripotent stem cells. More specifically, the present invention relates to a method for producing pancreatic progenitor cells, comprising causing the action of a factor having the inhibitory activity for cyclin-dependent kinase 8 and/or cyclin-dependent kinase 19 (hereinafter, also abbreviated to CDK8/19).

[Background Art]

Research is underway to induce the differentiation of pluripotent stem cells such as iPS cells or ES cells into pancreatic progenitor cells and to apply the obtained cells to the treatment of diabetes mellitus.

Heretofore, the induction of the differentiation of pluripotent stem cells into pancreatic progenitor cells has been practiced by performing, through several steps, the process of inducing the definitive endoderm from the pluripotent stem cells and inducing the pancreatic progenitor cells therefrom. For example, Fisk et al. disclose a method for inducing insulin-secreting cells by inducing the differentiation of human embryonic-stem cells in stages using activin A, cyclopamine, nicotinamide, and the like (Patent Literature 1).

Rezania et al. disclose a method for inducing differentiation into pancreatic progenitor cells, comprising the step of culturing cells expressing markers characteristic of pancreatic endocrine cells in a medium containing a sufficient amount of a cyclin-dependent kinase (CDK) inhibitor to increase the expression of MAFA (Patent Literature 2). MMFA is a mature pancreatic β cell marker, and the CDK inhibitor induces more matured pancreatic β cells from cells expressing markers characteristic of the pancreatic endocrine system. The literature states that the cyclin-dependent kinase (CDK) inhibitor used may inhibit CDK1, CDK2, CDK4, CDK5, and CDK9, but does not describe the inhibition of CDK8 or CDK19.

Citation List

Patent Literature

Patent Literature 1: US2008/0145889

Patent Literature 2: US9234178


Summary of Invention

Technical Problem

An object of the present invention is to provide a novel method for efficiently inducing pancreatic progenitor cells from pluripotent stem cells.

Solution to Problem

The inventors have found that the differentiation of NKX6.1-negative cells into NKX6.1-positive cells is induced by the action of a factor having CDK8/19-inhibiting activity in the process of inducing pancreatic progenitor cells from pluripotent stem cells; thus efficient induction of differentiation into pancreatic progenitor cells is achieved.

Specifically, the present invention provides the following [1] to [26]:

[1] a method for producing NKX6.1-positive cells, comprising culturing NKX6.1-negative cells in the presence of a factor having CDK8/19-inhibiting activity;

[1A] a method for producing NKX6.1-positive cells or a medicament comprising NKX6.1-positive cells, comprising culturing NKX6.1-negative cells in the presence of a factor having CDK8/19-inhibiting activity;
[2] the method according to [1], wherein the NKX6.1-negative cells are cultured in the presence of the factor having CDK8/19-inhibiting activity and a growth factor;
[3] the method according to [1] or [2], wherein the PDX-1-positive cells are enriched;
[4] the method according to any of [1] to [3], wherein the NKX6.1-positive cells are PDX-1-positive cells;
[5] a cell culture comprising human cells and a factor having CDK8/19-inhibiting activity, wherein at least about 10% of the human cells are NKX6.1-positive and PDX-1-positive cells;
[5A] a cell culture comprising human cells derived from human pluripotent stem cells and a factor having CDK8/19-inhibiting activity, wherein at least about 10% of the human cells are NKX6.1-positive and PDX-1-positive cells;
[6] the cell culture according to [5], wherein at least about 50% of the human cells are NKX6.1-positive and PDX-1-positive cells;
[7] the cell culture according to [5], wherein at least about 80% of the human cells are NKX6.1-positive and PDX-1-positive cells;
[8] human pluripotent stem cell-derived NKX6.1-positive and PDX-1-positive cells produced using a factor having CDK8/19-inhibiting activity;
[9] a medicament comprising human pluripotent stem cell-derived NKX6.1-positive and PDX-1-positive cells produced using a factor having CDK8/19-inhibiting activity;
[9A] the medicament according to [8] or [9], wherein the medicament is used for diabetes mellitus treatment;
[10] an inducer of differentiation into pancreatic progenitor cells, comprising a factor having CDK8/19-inhibiting activity;
[11] a medium for induction of differentiation into pancreatic progenitor cells, comprising a factor having CDK8/19-inhibiting activity;
[12] use of a factor having CDK8/19-inhibiting activity as an inducer of differentiation into pancreatic progenitor cells;
[13] compound 2 (4-((4-fluorophenyl)sulfonyl)-3-(2-(imidazo[1,2-b]pyridazin-6-ylsulfanyl)ethyl)-3,4-dihydroquinoxalin-2(1H)-one) or a salt thereof, compound 3 (2-(benzylamino)-4-(1H-pyrrolo[2,3-b]pyridin-3-yl)benzamide) or a salt thereof, compound 6 (N-butyl-8-(4-methoxyphenyl)-1,6-naphthyridine-2-carboxamide) or a salt thereof and compound 7 (8-(4-methylphenyl)-N,N-dipropyl-1,6-naphthyridine-2-carboxamide) or a salt thereof;
[14] compound 2 (4-((4-fluorophenyl)sulfonyl)-3-(2-(imidazo[1,2-b]pyridazin-6-ylsulfanyl)ethyl)-3,4-dihydroquinoxalin-2(1H)-one) or a salt thereof;
[15] compound 3 (2-(benzylamino)-4-(1H-pyrrolo[2,3-b]pyridin-3-yl)benzamide) or a salt thereof;
[16] compound 6 (N-butyl-8-(4-methoxyphenyl)-1,6-naphthyridine-2-carboxamide) or a salt thereof;
[17] compound 7 (8-(4-methylphenyl)-N,N-dipropyl-1,6-naphthyridine-2-carboxamide) or a salt thereof;
[18] the method according to any of [1] to [4], wherein the factor having CDK8/19-inhibiting activity is a compound selectively having CDK8/19-inhibiting activity among compounds described in US2012/0071477, WO2015/159937, WO2015/159938, WO2013/116786, WO2014/0038958, WO2014/134169, JP2015/506376, US2015/0274726, US2016/0000787, WO2016/009076, WO2016/0016951, WO2016/018511, WO2016/100782 and WO2016/182904;
[19] the method according to any of [1] to [4], wherein the NKX6.1-positive cells are induced from the NKX6.1-negative cells by culture in the presence of the factor having CDK8/19-inhibiting activity at the stage of the differentiation of posterior foregut cells into pancreatic progenitor cells;
[20] the method according to any of [1] to [4], [18], and
[19], wherein the medium contains a serum replacement and an antibiotic;
[21] the method according to any of [1] to [4], and [18] to [20], wherein the serum replacement is selected from B-27 supplement, KSR, StemSure Serum Replacement, and ITS-G;
[22] the method according to [20] or [21], wherein the antibiotic is selected from Antibiotic-Antimycotic, penicillin, streptomycin, and mixtures thereof;
[23] a cell culture comprising a factor having CDK8/19-inhibiting activity and 70% or more of NKX6.1-positive cells;
[24]a cell population enriched for NKX6.1-positive cells, the cell population being produced by a method according to any of [1] to [4], and [18] to [22];
[25] a cell population comprising 70% or more of NKX6.1-positive cells, the cell population being produced by a method according to any of [1] to [4], and [18] to [22]; and
[26] a pharmaceutical composition comprising a cell population according to [25].

Advantageous Effects of Invention

According to the present invention, pancreatic progenitor cells may be efficiently induced by promoting the differentiation of NKX6.1-negative cells into NKX6.1-positive cells in the process of inducing the differentiation of pluripotent stem cells into pancreatic progenitor cells.

Brief Description of Drawings

[Figure 1] Figure 1 shows compounds (compounds 1 to 7) having CDK8-inhibiting activity and CDK19-inhibiting activity and having various chemical structures.

[Figure 2] Figure 2 shows the proportion (%) of NKX6.1-positive and PDX-1-positive cells. Figure 2A shows the case of using a medium containing a ROCK inhibitor (KENY50-KENT), and Figure 2B shows the case of using a medium containing no ROCK inhibitor (KEN-KENT). The graph shows, from the left, iPS cells, cells before culture at stage 4 (s3d2: stage 3, day 2), cells after culture at stage 4 (without the addition of a CDK8/19 inhibitor (negative control), and cells after culture at stage 4 (with various CDK8 and CDK19 inhibitors).

[Figure 3] Figure 3 shows the proportion (%) of NKX6.1-positive and PDX-1-positive cells. In Figure 3, CDK1/2 shows a commercially available CDK1/2 inhibitor CAS443798-55-8 (217714, Merck).

Description of Embodiments

1. Terminology

As used herein, "about" or "around" refers to a value which may vary up to plus or minus 25%, 20%, 10%, 8%, 6%, 5%, 4%, 3%, 2%, or 1% from the reference value. Preferably, the term "about" or "around" refers to a range from minus or plus 15%, 10%, 5%, or 1% from the reference value.

As used herein, "substantially" or "essentially" refers to a value equal to or greater than 90%, preferably 95%, 96%, 97%, 98%, or 99% of the reference value. "Substantially identical" or "essentially identical" means an identity equal to or greater than 90%, preferably 95%, 96%, 97%, 98%, or 99% with the reference value and "substantially free of" or" essentially free of" means that a certain substance is contained at a content of not more than 5%, preferably not more than 4%, 3%, 2%,1% or undetectable.

As used herein, "comprise(s)" or "comprising" means inclusion of the element(s) following the word without limitation thereto. Accordingly, it indicates inclusion of the element(s) following the word, but does not indicate exclusion of any other element.

As used herein, "consist(s) of" or "consisting of" means inclusion of all the element(s) following the phrase and limitation thereto. Accordingly, the phrase "consist(s) of" or "consisting of" indicates that the enumerated element(s) is required or essential and substantially no other elements exist. "Consist(s) essentially of" or "consisting essentially of" means inclusion of any element following the phrase and limitation of other elements to those that do not affect the activity or effect of the enumerated element(s) specified in the disclosure. Accordingly, the phrase "consist(s) essentially of" or "consisting essentially of" indicates that the enumerated element(s) is required or essential, but other elements are optional and may exist or not exist depending on whether they affect the activity or effect of the enumerated element(s).

As used herein, "promote", "enhance", or "increase" refers generally to generating a response larger than a physiological response caused by a vehicle or control. For example, a "promoted", "enhanced", or "increased" response is preferably a statistically significant response and can be equal to or greater than 1.1 times, 1.2 times, 1.5 times, twice, 3 times, 4 times, 5 times, 6 times, 7 times, 8 times, 9 times, 10 times, 15 times, 20 times, 30 times of the response generated by a vehicle or control.

As used herein, "lower", "decrease", or "attenuate" refers generally to generating a response lower than a response caused by a vehicle or control. "Lowered", "decreased", or "attenuated" response is preferably a statistically significant response and can be equal to or lower than 1/1.1, 1/1.2, 1/1.5, 1/2, 1/3, 1/4, 1/5, 1/6, 1/7, 1/8, 1/9, 1/10, 1/15, 1/20, 1/30 of a response generated by a vehicle or control.

As used herein, "ex vivo" is used to refer generally to an experiment or measurement conducted in living tissue in an artificial environment out of the living body, such as cultured tissue and cultured cells. Tissue or cells to be used may be frozen for preservation and may be thawed for subsequent treatment out of the living body. The term "in vitro" is used when a tissue culture experiment of living cells or living tissue is conducted for a few days or more continuously. "In vitro" may be used interchangeably with "ex vivo". In contrast, the term "in vivo" is used to refer generally to a phenomenon occurring in the living body, such as proliferation of cells.

As used herein, "containing no feeder (cells)" or "feeder free" means basically containing no feeder cells and using no medium preconditioned by culturing feeder cells. Accordingly, the medium does not contain any substance, such as a growth factor or a cytokine, secreted by feeder cells.

"Feeder cells" or "feeder" means cells that are co-cultured with another kind of cells, support the cells, and provide an environment that allows the cells to grow. The feeder cells may be derived from the same species as or a different species from the cells that they support. For example, as a feeder for human cells, human skin fibroblasts or human embryonic-stem cells may be used or a primary culture of murine embryonic fibroblasts or immortalized murine

embryonic fibroblasts may be used. The feeder cells can be inactivated by exposure to radiation or treatment with mitomycin C.

As used herein, "adhered (adherent)" refers to cells are attached to a container, for example, cells are attached to a cell culture dish or a flask made of a sterilized plastic (or coated plastic) in the presence of an appropriate medium. Some cells cannot be maintained or grow in culture without adhering to the cell culture container. In contrast, non-adherent cells can be maintained and proliferate in culture without adhering to the container.

As used herein, "culture" refers to maintaining, growing, and/or differentiating cells in in vitro environment. "Culturing" means maintaining, proliferating (growing), and/or differentiating cells out of tissue or the living body, for example, in a cell culture dish or flask.

As used herein, "enrich(es)" and "enrichment" refer to increasing the amount of a certain component in a composition such as a composition of cells and "enriched" refers, when used to describe a composition of cells, for example, a cell population, to a cell population increased in the amount of a certain component in comparison with the percentage of such component in the cell population before the enrichment. For example, a composition such as a cell population can be enriched for a target cell type and, accordingly, the percentage of the target cell type is increased in comparison with the percentage of the target cells present in the cell population before the enrichment. A cell population can be enriched for a target cell type by a method of selecting and sorting cells known in the art. A cell population can be enriched by a specific process of sorting or selection described herein. In a certain embodiment of the present invention, a cell population is enriched for a target cell population at least 20%, 30%, 40%, 50%, 60%, 70%, 80%, 85%, 90%, 95%, 97%, 98%, or 99% by a method of enriching the target cell population.

For example, culture for 4 days with the addition of a factor having CDK8/19-inhibiting activity at stage 4 enriches NKX6.1-positive cells or/and PDX-1-positive cells, enhances the efficiency of production, and enables preparation of more safer cells.

As used herein, "deplete(s)" and "depletion" refer to decreasing the amount of a certain component in a composition such as a composition of cells and "depleted" refers, when used to describe a composition of cells, for example, a cell population, to a cell population decreased in the amount of a certain component in comparison with the percentage of such component in the cell population before the depletion. For example, a composition such as a cell population can be depleted for a target cell type and, accordingly, the percentage of the target cell type is decreased in comparison with the percentage of the target cells present in the cell population before the depletion. A cell population can be depleted for a target cell type by a method of selecting and sorting cells known in the art. A cell population can be depleted by a specific process of sorting or selection described herein. In a certain embodiment of the present invention, a cell population is reduced (depleted) for a target cell population at least 50%, 80%, 85%, 90%, 95%, 97%, 98%, or 99% by a method of depleting a target cell population.

As used herein, "purify(ies)" and "purification" refer to removing impurities in a composition such as a composition of cells and making it pure for a certain component and "purified" refers, when used to describe a composition of cells, for example, a cell population, to a cell population in which the amount of impurities is decreased in comparison with the percentage of such components in the cell population before purification and the purity of a certain component is improved. For example, a composition such as a cell population can be purified for a target cell type and, accordingly, the percentage of the target cell type is increased in comparison with the percentage of the target cells present in the cell population before the purification. A cell population can be purified for a target cell type by a method of selecting and sorting cells known in the art. A cell population can be purified by a specific process of sorting or selection described herein. In a certain embodiment of the present invention, the purity of a target cell population is brought by a method of purifying a target cell population to at least 70%, 80%, 85%, 90%, 95%, 97%, 98%, or 99% or to the extent at which impurities (including contaminant cells) are undetectable.

As used herein, "isolated" refers to being separated from the original environment. For example, "isolated cell population" refers to a cell population separated from a natural cell environment in vitro or out of the living body and from other components of tissue or an organ.

As used herein, "marker" means a cell antigen that is specifically expressed on cell surface, in cytosol, and/or in nucleus of a predetermined cell type or a gene thereof, such as "marker protein" and "marker gene". Preferably, a marker is a cell surface marker and this allows concentration, isolation, and/or detection of living cells. A marker can be a positive selection marker or a negative selection marker.

The detection of a marker protein can be conducted by an immunological assay, for example, ELISA, immunostaining, or flow cytometry using an antibody specific for the marker protein. As the antibody specific for the marker protein, an antibody that binds to a specific amino acid sequence of the marker protein or a specific sugar chain on the marker protein can be used. In case of an intracellularly expressed marker protein which does not appear on the surface of cells (for example, a transcription factor or a subunit thereof), the marker protein of interest can be detected by expressing the marker protein with a reporter protein and detecting the reporter protein. This approach may be preferably used when an appropriate cell surface marker is not found. The detection of a marker gene can be conducted by a method of amplifying and/or detecting nucleic acid known in the art, for example, RT-PCR, microarray, biochip, or the like.

As used herein, "positive" for a marker protein or gene means being expressed in an amount detectable by an approach known in the art. The detection of a protein can be conducted by an immunological assay, for example, ELISA, immunostaining, or flow cytometry using an antibody. In case of an intracellularly expressed protein which does not appear on the surface of cells (for example, a transcription factor or a subunit thereof), the protein of interest can be detected by expressing the protein with a reporter protein and detecting the reporter protein. "Negative" for a marker protein or gene means that the expression level of a protein or a gene is less than the lower detection limit in all or any of known approaches such as RT-PCR, microarray, and biochip. A PDX1-positive marker and a NKX6.1-positive marker can be detected by a method known per se (for example, flow cytometry).

As used herein, "expression" is defined as transcription and/or translation of a certain nucleotide sequence driven by an intracellular promoter.

As used herein, "autologous" refers to cells derived from the same subject. "Allogenic" refers to cells of the same species that are genetically different from the cells to be compared. "Isogeneic" refers to cells of a different subject that are genetically same as the cells to be compared. "Xenogeneic" refers to cells of a species different from the cells to be compared.

As used herein, "pluripotency" means the ability to differentiate into tissues and cells having various different shapes and functions and to differentiate into cells of any lineage of the 3 germ layers. "Pluripotency" is different from "totipotency", which is the ability to differentiate into any tissue of the living body, including the placenta, in that pluripotent cells cannot differentiate into the placenta and therefore, do not have the ability to form an individual.

As used herein, "multipotency" means the ability to differentiate into plural and limited numbers of linages of cells. For example, mesenchymal stem cells, hematopoietic stem cells, neural stem cells are multipotent, but not pluripotent.

"Pluripotent stem cells" refers to embryonic-stem cells (ES cells) and cells potentially having a pluripotency similar to that of ES cells, that is, the ability to differentiate into various tissues (all of the endodermal, mesodermal, and ectodermal tissues) in the living body. Examples of cells having a pluripotency similar to that of ES cells include "induced pluripotent stem cells" (that may be herein also referred to as "iPS cells").

As used herein, human cells include human pluripotent stem cells.

Human cells are preferably human cells derived from human pluripotent stem cells.

Available "ES cells" include murine ES cells, such as various murine ES cell lines established by inGenious, RIKEN, and the like, and human ES cells, such as various human ES cell lines established by NIH, RIKEN, Kyoto University, Cellartis, and the like. For example, available ES cell lines include CHB-1 to CHB-12, RUES1, RUES2, HUES1 to HUES28 distributed by NIH, and the like; H1 and H9 distributed by WisCell Research; and KhES-1, KhES-2, KhES-3, KhES-4, KhES-5, SSES1, SSES2, SSES3 distributed by RIKEN, and the like.

"Induced pluripotent stem cells" refers to cells that are obtained by reprograming mammalian somatic cells or undifferentiated stem cells by introducing particular factors (nuclear reprogramming factors). At present, there are various "induced pluripotent stem cells" and iPS cells established by Yamanaka, et al. by introducing the 4 factors Oct3/4, Sox2, Klf4, c-Myc into murine fibroblasts (Takahashi K, Yamanaka S., Cell, (2006) 126: 663-676); iPS cells derived from human cells, established by introducing similar 4 factors into human fibroblasts (Takahashi K, Yamanaka S., et al. Cell, (2007) 131: 861-872.); Nanog-iPS cells established by sorting cells using expression of Nanog as an indicator after introduction of the 4 factors (Okita, K., Ichisaka, T., and Yamanaka, S. (2007). Nature 448, 313-317.); iPS cells produced by a method not using c-Myc (Nakagawa M, Yamanaka S., et al. Nature Biotechnology, (2008) 26, 101-106); and iPS cells established by introducing 6 factors in a virus-free way (Okita K et al. Nat. Methods 2011 May; 8(5): 409-12, Okita K et al. Stem Cells. 31 (3) 458-66) may be also used. Also, induced pluripotent stem cells established by introducing the 4 factors OCT3/4, SOX2, NANOG, and LIN28 by Thomson et al. (Yu J., Thomson JA. et al., Science (2007) 318: 1917-1920.); induced pluripotent stem cells produced by Daley et al. (Park IH, Daley GQ.et al., Nature (2007) 451: 141-146); induced pluripotent stem cells produced by Sakurada et al. (Japanese Unexamined Patent Application Publication No. 2008-307007) and the like may be used.

In addition, any of known induced pluripotent stem cells known in the art described in all published articles (for example, Shi Y., Ding S., et al., Cell Stem Cell, (2008) Vol 3, Issue 5, 568-574; Kim JB., Scholer HR., et al., Nature, (2008) 454, 646-650; Huangfu D., Melton, DA., et al., Nature Biotechnology, (2008) 26, No. 7, 795-797) or patents (for example, Japanese Unexamined Patent Application Publication No. 2008-307007, Japanese Unexamined Patent Application Publication No. 2008-283972, US2008-2336610, US2009-047263, WO2007-069666, WO2008-118220, WO2008-124133, WO2008-151058, WO2009-006930, WO2009-006997, WO2009-007852) may be used.

Available induced pluripotent cell lines include various iPS cell lines established by NIH, Institute of Physical and Chemical Research (RIKEN), Kyoto University and the like. For example, such human iPS cell lines include the RIKEN cell lines HiPS-RIKEN-1A, HiPS-RIKEN-2A, HiPS-RIKEN-12A, and Nips-B2 and the Kyoto University cell lines Ff-WJ-18, Ff-I01s01, Ff-I01s02, Ff-I01s04, Ff-I01s06, Ff-I14s03, Ff-I14s04, QHJI01s01, QHJI01s04, QHJI14s03, QHJI14s04, 253G1, 201B7, 409B2, 454E2, 606A1, 610B1, 648A1, CDI cell lines MyCell iPS Cells (21525.102.10A), MyCell iPS Cells (21526.101.10A), and the like.

"Definitive endoderm cells" are cells that are derived from mesendoderm cells and are capable of differentiating into the liver, the pancreas, the lung, or the small intestine system in the future. The definitive endoderm cells are usually characterized by SOX17 positivity, CXCR4 positivity, FOXA2 positivity, and the like. The definitive endoderm cells are induced to differentiate into various endodermal cells using various induction factors. Accordingly, the induction of the definitive endoderm may be regarded as an initial step for inducing endodermal cells from human pluripotent stem cells. A plurality of methods for inducing the definitive endoderm have been reported, and a commercially available kit may be utilized.

As used herein, "factor having CDK8/19-inhibiting activity" means any substance having the inhibitory activity for CDK8/19. CDK8, in contrast to the other proteins of the same CDK family, is not required for cell proliferation. The inhibition of CDK8 has no great effect under usual conditions. CDK19 and CDK8 are similar to each other as mentioned above. Usually, the inhibition of CDK8 also involves the inhibition of CDK19.

As used herein, a method for producing NKX6.1-positive cells, comprising culturing NKX6.1-negative cells in the presence of a factor having CDK8/19-inhibiting activity is carried out *in vitro* or/and *in vivo.*

"Growth factors" are endogenous proteins that promote differentiation and/or proliferation of particular cells. Examples of "growth factors" include epidermal growth factor (EGF), acid fibroblast growth factor (aFGF), basic fibroblast growth factor (bFGF), hepatocyte growth factor (HGF), insulin-like growth factor 1 (IGF-1), insulin-like growth factor 2 (IGF-2), keratinocyte growth factor (KGF), nerve growth factor (NGF), platelet-derived growth factor (PDGF), transformation growth factor beta (TGF-β), vascular endothelial growth factor (VEGF), transferrin, various interleukins (for example, IL-1 to IL-18), various colony-stimulating factors (for example, granulocyte/macrophage colony-stimulating factor (GM-CSF)), various interferons (for example, IFN-γ, and the like), and other cytokines having effects on stem cells, for example, stem cell factor (SCF), and erythropoietin (Epo) .

"ROCK inhibitors" means substances that inhibit Rho kinase (ROCK: Rho-associated, coiled-coil containing protein kinase) and may be substances that inhibit either of ROCK I and ROCK II. The ROCK inhibitors are not particularly limited as long as they have the aforementioned function and examples include N-(4-pyridinyl)-4β-[(R)-1-aminoethyl]cyclohexane-1α-carboxamide (that may be herein also referred to as Y-27632), Fasudil (HA1077), (2S)-2-methyl-1-[(4-methyl-5-isoquinolinyl]sulfonyl]hexahydro-1H-1,4-diazepine (H-1152), 4β-[(1R)-1-aminoethyl]-N-(4-pyridyl)benzene-1α-carboxamide (Wf-536), N-(1H-pyrrolo[2,3-b]pyridin-4-yl)-4PER(R)-1-aminoethyl]cyclohexane-1α-carboxamide (Y-30141), N-(3-{[2-(4-amino-1,2,5-oxadiazol-3-yl)-1-ethyl-1H-imidazo[4,5-c]pyridin-6-yl]oxy}phenyl)-4-{[2-(4-morpholinyl)ethyl]-oxy}benzamide (GSK269962A), N-(6-fluoro-1H-indazol-5-yl)-6-methyl-2-oxo-4-[4-(trifluoromethyl)phenyl]-3,4-dihydro-1H-pyridine-5-carboxamide (GSK429286A). The ROCK inhibitors are not limited to these and antisense oligonucleotides and siRNA to ROCK mRNA, antibodies that bind to ROCK, and dominant negative ROCK mutants can also be used, commercially available, or synthesized according to a known method as ROCK inhibitors.

"GSK3β inhibitors" are substances having the inhibitory activity for GSK3β (glycogen synthase kinase 3β). GSK3 (glycogen synthase kinase 3) is a serine/threonine protein kinase and involved in many signaling pathways associated with the production of glycogen, apoptosis, maintenance of stem cells, etc. GSK3 has the 2 isoforms α and β. "GSK3β inhibitors" used in the present invention are not particularly limited as long as they have the GSK3β-inhibiting activity and they may be substances having both the GSK3α-inhibiting activity and the GSK3β-inhibiting activity.

Examples of GSK3β inhibitors include CHIR98014 (2-[[2-[(5-nitro-6-aminopyridin-2-yl)amino]ethyl]amino]-4-(2,4-dichlorophenyl)-5-(1H-imidazol-1-yl)pyrimidine), CHIR99021 (6-[[2-[[4-(2,4-dichlorophenyl)-5-(4-methyl-1H-imidazol-2-yl)-2-pyrimidinyl]amino]ethyl]amino]nicotinonitrile), TDZD-8 (4-benzyl-2-methyl-1,2,4-thiadiazolidine-3,5-dione), SB216763 (3-(2,4-dichlorophenyl)-4-(1-methyl-1H-indol-3-yl)-1H-pyrrole-2,5-dione), TWS-119 (3-[6-(3-aminophenyl)-7H-pyrrolo[2,3-d]pyrimidin-4-yloxy]phenol), kenpaullone, 1-azakenpaullone, SB216763 (3-(2,4-dichlorophenyl)-4-(1-methyl-1H-indol-3-yl)-1H-pyrrole-2,5-dione), SB415286 (3-[(3-chloro-4-hydroxyphenyl)amino]-4-(2-nitrophenyl)-1H-pyrrole-2,5-dione), and AR-AO144-18, CT99021, CT20026, BIO, BIO-acetoxime, pyridocarbazole-ruthenium cyclopentadienyl complex, OTDZT, alpha-4-dibromoacetophenone, lithium, and the like. GSK3β is not limited to these and antisense oligonucleotides and siRNA to GSK3β mRNA, antibodies that bind to GSK3β, dominant negative GSK3β mutants, and the like can also be used, commercially available, or synthesized according to a known method as GSK3β inhibitors.

In the present invention, "serum replacement" is preferably used instead of serum. Examples of "serum replacement" include Knockout Serum Replacement (KSR: Invitrogen), StemSure Serum Replacement (Wako), B-27 supplement, N2-supplement, albumin (for example, lipid rich albumin), insulin, transferrin, fatty acids, collagen precursors, trace elements (for example, zinc, selenium (for example, sodium selenite)), 2-mercaptoethanol, 3'-thiolglycerol, or mixtures thereof (for example, ITS-G). Preferred serum replacements are B-27 supplement, KSR, StemSure Serum Replacement, ITS-G. The concentration of serum replacement in a medium when added into a medium is 0.01-10% by weight, and preferably 0.1-2% by weight.

2. Method for producing NKX6.1-positive cells

The present invention provides a method for producing NKX6.1-positive cells, comprising culturing NKX6.1-negative cells in the presence of a factor having CDK8/19-inhibiting activity.
The factor having CDK8/19-inhibiting activity acts on NKX6.1-negative cells to induce NKX6.1-positive cells. In other words, the factor having CDK8/19-inhibiting activity enriches NKX6.1-positive cells.
The "factor having CDK8/19-inhibiting activity" used in the present invention is not particularly limited as long as it has the inhibitory activity for CDK8/19. Any factor that directly or indirectly inhibits the functions of CDK8/19 can be used. The "factor having CDK8/19-inhibiting activity" specifically refers to a factor inhibiting 50% or more of CDK8/19. A method for examining the presence or absence of CDK8/19-inhibiting activity may be selected from known methods. Examples thereof include a method of Example 1 described herein. The "factor having CDK8/19-inhibiting activity" can be found in patent literatures or non patent literatures. Specific examples of the "factor having CDK8/19-inhibiting activity" include compounds having CDK8/19-inhibiting activity (or salts thereof) among compounds described in US2012/0071477, WO2015/159937, WO2015/159938, WO2013/116786, WO2014/0038958, WO2014/134169, JP2015/506376, US2015/0274726, US2016/0000787, WO2016/009076, WO2016/0016951, WO2016/018511, WO2016/100782 and WO2016/182904. More specific examples thereof include compounds having the inhibitory activity selective for CDK8/19 (or salts thereof) among the compounds.
Examples of the "factor having CDK8/19-inhibiting activity" include compounds having the inhibitory activity selective for CDK8/19 (or salts thereof) among compounds having a structural formula represented by the following general formula, and specifically include compounds inhibiting 50% or more of CDK8/19 (or salts thereof) among the compounds having a structural formula represented by the following general formula.
Examples of the compound having CDK8/19-inhibiting activity include compounds represented by the formula:

[Formula 1]

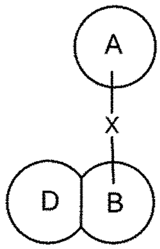

(I)

wherein ring A shows benzene optionally having substituent(s) or a heterocycle optionally having substituent(s) (ring A may form a fused ring optionally having substituent(s));
ring BD shows naphthalene optionally having substituent(s) or bi or tricyclic heterocycles optionally having substituent(s) (ring BD may further form a fused ring optionally having substituent(s)); and
X shows a linker

or salts thereof.
Ring A shows benzene optionally having substituent(s) or a heterocycle optionally having substituent(s) (ring A may form a fused ring).
Examples of the "heterocycle" of the "heterocycle optionally having substituent(s)" shown as ring A include aromatic heterocycles and non-aromatic heterocycles each containing, as a ring-constituting atom besides carbon atom, 1 to 4 hetero atoms selected from a nitrogen atom, a sulfur atom and an oxygen atom.
Examples of the "aromatic heterocycle" include a 5-to 6-membered aromatic heterocycle containing, as a ring-constituting atom besides carbon atom, 1 to 4 hetero atoms selected from a nitrogen atom, a sulfur atom and an oxygen atom. Preferable examples of the "aromatic heterocycle" include 5- or 6-membered monocyclic aromatic heterocycles such as thiophene, furan, pyrrole, imidazole, pyrazole, thiazole, isothiazole, oxazole, isoxazole, pyridine, pyrazine, pyrimidine, pyridazine, 1,2,4-oxadiazole, 1,3,4-oxadiazole, 1,2,4-thiadiazole, 1,3,4-thiadiazole, triazole, tetrazole, triazine.
Examples of the "non-aromatic heterocycle" include a 3- to 14-membered (preferably 4- to 10-membered) non-aromatic heterocycle containing, as a ring-constituting atom besides carbon atom, 1 to 4 hetero atoms selected from a nitrogen atom, a sulfur atom and an oxygen atom. Preferable examples of the "non-aromatic heterocycle" include 3- to 8-membered monocyclic non-aromatic heterocycles such as aziridine, oxirane, thiirane, azetidine, oxetane, thietane, tetrahydrothiophene, tetrahydrofuran, pyrroline, pyrrolidine, imidazoline, imidazolidine, oxazoline, oxazolidine, pyrazoline, pyrazolidine, thiazoline, thiazolidine, tetrahydroisothiazole, tetrahydrooxazole, tetrahydroisoxazole, piperidine, piperazine, tetrahydropyridine, dihydropyridine, dihydrothiopyran, tetrahydropyrimidine, tetrahydropyridazine, dihydropyran, tetrahydropyran, tetrahydrothiopyran, morpholine, thiomorpholine, azepanine, diazepane, azepine, azocane, diazocane, oxepane.

Ring A may form a fused ring optionally having substituent(s).

Examples of the "fused ring" of the "fused ring optionally having substituent(s)" shown as ring A include aromatic hydrocarbon ring such as naphthalene; 8- to 14-membered fused polycyclic (preferably bi or tricyclic) aromatic heterocycles such as benzothiophene, benzofuran, benzimidazole, benzoxazole, benzisoxazole, benzothiazole, benzisothiazole, benzotriazole, imidazopyridine, thienopyridine, furopyridine, pyrrolopyridine, pyrazolopyridine, oxazolopyridine, thiazolopyridine, imidazopyrazine, imidazopyrimidine, thienopyrimidine, furopyrimidine, pyrrolopyrimidine, pyrazolopyrimidine, oxazolopyrimidine, thiazolopyrimidine, pyrazolopyrimidine, pyrazolotriazine, naphtho[2,3-b]thiophene, phenoxathiin, indole, isoindole, 1H-indazole, purine, isoquinoline, quinoline, phthalazine, naphthyridine, quinoxaline, quinazoline, cinnoline, carbazole, β-carboline, phenanthridine, acridine, phenazine, phenothiazine, phenoxazine; 9- to 14-membered fused polycyclic (preferably bi or tricyclic) non-aromatic heterocycles such as dihydrobenzofuran, dihydrobenzimidazole, dihydrobenzoxazole, dihydrobenzothiazole, dihydrobenzisothiazole, dihydronaphtho[2,3-b]thiophene, tetrahydroisoquinoline, tetrahydroquinoline, 4H-quinolizine, indoline, isoindoline, tetrahydrothieno[2,3-c]pyridine, tetrahydrobenzazepine, tetrahydroquinoxaline, tetrahydrophenanthridine, hexahydrophenothiazine, hexahydrophenoxazine, tetrahydrophthalazine, tetrahydronaphthyridine, tetrahydroquinazoline, tetrahydrocinnoline, tetrahydrocarbazole, tetrahydro-β-carboline, tetrahydroacridine, tetrahydrophenazine, tetrahydrothioxanthene, octahydroisoquinoline.

Ring BD shows naphthalene optionally having substituent(s) or bi or tricyclic heterocycles optionally having substituent(s) (ring BD may form a fused ring optionally having substituent(s)).

Examples of the "bi or tricyclic heterocycles" of the "bi or tricyclic heterocycles optionally having substituent(s)" shown as ring BD include 8- to 14-membered heterocycles containing, as a ring-constituting atom besides carbon atom, 1 to 4 hetero atoms selected from a nitrogen atom, a sulfur atom and an oxygen atom. Preferable examples of the "bi or tricyclic heterocycles" include 8- to 14-membered fused polycyclic (preferably bi or tricyclic) aromatic heterocycles such as benzothiophene, benzofuran, benzimidazole, benzoxazole, benzisoxazole, benzothiazole, benzisothiazole, benzotriazole, imidazopyridine, thienopyridine, furopyridine, pyrrolopyridine, pyrazolopyridine, oxazolopyridine, thiazolopyridine, imidazopyrazine, imidazopyrimidine, thienopyrimidine, furopyrimidine, pyrrolopyrimidine, pyrazolopyrimidine, oxazolopyrimidine, thiazolopyrimidine, pyrazolopyrimidine, pyrazolotriazine, naphtho[2,3-b]thiophene, phenoxathiin, indole, isoindole, 1H-indazole, purine, isoquinoline, quinoline, phthalazine, naphthyridine, quinoxaline, quinazoline, cinnoline, carbazole, β-carboline, phenanthridine, acridine, phenazine, phenothiazine, phenoxazine; 9- to 14-membered fused polycyclic (preferably bi or tricyclic) non-aromatic heterocycles such as dihydrobenzofuran, dihydrobenzimidazole, dihydrobenzoxazole, dihydrobenzothiazole, dihydrobenzisothiazole, dihydronaphtho[2,3-b]thiophene, tetrahydroisoquinoline, tetrahydroquinoline, 4H-quinolizine, indoline, isoindoline, tetrahydrothieno[2,3-c]pyridine, tetrahydrobenzazepine, tetrahydroquinoxaline, tetrahydrophenanthridine, hexahydrophenothiazine, hexahydrophenoxazine, tetrahydrophthalazine, tetrahydronaphthyridine, tetrahydroquinazoline, tetrahydrocinnoline, tetrahydrocarbazole, tetrahydro-β-carboline, tetrahydroacridine, tetrahydrophenazine, tetrahydrothioxanthene, octahydroisoquinoline.

Ring BD may further form a fused ring optionally having substituent(s).

Examples of the "fused ring" of the "fused ring optionally having substituent(s)" include rings formed by fusing an aromatic hydrocarbon ring such as naphthalene, 8- to 14-membered fused polycyclic (preferably bi or tricyclic) aromatic heterocycles, or 9- to 14-membered fused polycyclic (preferably bi or tricyclic) non-aromatic heterocycles to benzene or the aforementioned monocyclic heterocycle (5- to 6-membered monocyclic aromatic heterocycle or 4- to 8-membered monocyclic non-aromatic heterocycles).

Examples of linker shown as X include a bond, -O-, - NH-, $C_{1-6}$ alkyl optionally having -N- substituent(s), -S-, -SO-, $-SO_2-$, alkylene (preferably $C_{1-13}$ alkylene) optionally having a substituent(s), alkenylene (preferably $C_{2-13}$ alkylene) optionally having a substituent(s), wherein -C- in the alkylene or the alkenylene may be substituted by -O-, -N- or -S-. The position substituted by -O-, -N- or -S- may be at the terminal or in the chain of the alkylene or the alkenylene. Examples of the substituents of the "alkylene" or the "alkenylene" include $C_{1-6}$ alkyl, oxo, $C_{6-14}$ aryl (such as phenyl). The number of substituents may be 1 to 3.

In the present specification, examples of the "substituent" include a halogen atom, a cyano group, a nitro group, an optionally substituted hydrocarbon group, an optionally substituted heterocyclic group, an acyl group, an optionally substituted amino group, an optionally substituted carbamoyl group, an optionally substituted thiocarbamoyl group, an optionally substituted sulfamoyl group, an optionally substituted hydroxy group, an optionally substituted sulfanyl (SH) group and an optionally substituted silyl group.

In the present specification, examples of the "hydrocarbon group" (including "hydrocarbon group" of "optionally substituted hydrocarbon group") include a $C_{1-6}$ alkyl group, a $C_{2-6}$ alkenyl group, a $C_{2-6}$ alkynyl group, a $C_{3-10}$ cycloalkyl group, a $C_{3-10}$ cycloalkenyl group, a $C_{6-14}$ aryl group and a $C_{7-16}$ aralkyl group.

In the present specification, examples of the "optionally substituted hydrocarbon group" include a hydrocarbon group optionally having substituent(s) selected from the following substituent group A.

[substituent group A]

(1) a halogen atom,
(2) a nitro group,
(3) a cyano group,
(4) an oxo group,
(5) a hydroxy group,
(6) an optionally halogenated $C_{1-6}$ alkoxy group,
(7) a $C_{6-14}$ aryloxy group (e.g., phenoxy, naphthoxy),
(8) a $C_{7-16}$ aralkyloxy group (e.g., benzyloxy),
(9) a 5- to 14-membered aromatic heterocyclyloxy group (e.g., pyridyloxy),
(10) a 3- to 14-membered non-aromatic heterocyclyloxy group (e.g., morpholinyloxy, piperidinyloxy),
(11) a $C_{1-6}$ alkyl-carbonyloxy group (e.g., acetoxy, propanoyloxy),
(12) a $C_{6-14}$ aryl-carbonyloxy group (e.g., benzoyloxy, 1-naphthoyloxy, 2-naphthoyloxy),
(13) a $C_{1-6}$ alkoxy-carbonyloxy group (e.g., methoxycarbonyloxy, ethoxycarbonyloxy, propoxycarbonyloxy, butoxycarbonyloxy),
(14) a mono- or di-$C_{1-6}$ alkyl-carbamoyloxy group (e.g., methylcarbamoyloxy, ethylcarbamoyloxy, dimethylcarbamoyloxy, diethylcarbamoyloxy),
(15) a $C_{6-14}$ aryl-carbamoyloxy group (e.g., phenylcarbamoyloxy, naphthylcarbamoyloxy),
(16) a 5- to 14-membered aromatic heterocyclylcarbonyloxy group (e.g., nicotinoyloxy),
(17) a 3- to 14-membered non-aromatic heterocyclylcarbonyloxy group (e.g., morpholinylcarbonyloxy, piperidinylcarbonyloxy),
(18) an optionally halogenated $C_{1-6}$ alkylsulfonyloxy group (e.g., methylsulfonyloxy, trifluoromethylsulfonyloxy),
(19) a $C_{6-14}$ arylsulfonyloxy group optionally substituted by a $C_{1-6}$ alkyl group (e.g., phenylsulfonyloxy, toluenesulfonyloxy),
(20) an optionally halogenated $C_{1-6}$ alkylthio group,
(21) a 5- to 14-membered aromatic heterocyclic group,
(22) a 3- to 14-membered non-aromatic heterocyclic group,
(23) a formyl group,
(24) a carboxy group,
(25) an optionally halogenated $C_{1-6}$ alkyl-carbonyl group,
(26) a $C_{6-14}$ aryl-carbonyl group,
(27) a 5- to 14-membered aromatic heterocyclylcarbonyl group,
(28) a 3- to 14-membered non-aromatic heterocyclylcarbonyl group,
(29) a $C_{1-6}$ alkoxy-carbonyl group,
(30) a $C_{6-14}$ aryloxy-carbonyl group (e.g., phenyloxycarbonyl, 1-naphthyloxycarbonyl, 2-naphthyloxycarbonyl),
(31) a $C_{7-16}$ aralkyloxy-carbonyl group (e.g., benzyloxycarbonyl, phenethyloxycarbonyl),
(32) a carbamoyl group,
(33) a thiocarbamoyl group,
(34) a mono- or di-$C_{1-6}$ alkyl-carbamoyl group,
(35) a $C_{6-14}$ aryl-carbamoyl group (e.g., phenylcarbamoyl),
(36) a 5- to 14-membered aromatic heterocyclylcarbamoyl group (e.g., pyridylcarbamoyl, thienylcarbamoyl),
(37) a 3- to 14-membered non-aromatic heterocyclylcarbamoyl group (e.g., morpholinylcarbamoyl, piperidinylcarbamoyl),
(38) an optionally halogenated $C_{1-6}$ alkylsulfonyl group,
(39) a $C_{6-14}$ arylsulfonyl group,
(40) a 5- to 14-membered aromatic heterocyclylsulfonyl group (e.g., pyridylsulfonyl, thienylsulfonyl),
(41) an optionally halogenated $C_{1-6}$ alkylsulfinyl group,
(42) a $C_{6-14}$ arylsulfinyl group (e.g., phenylsulfinyl, 1-naphthylsulfinyl, 2-naphthylsulfinyl),
(43) a 5- to 14-membered aromatic heterocyclylsulfinyl group (e.g., pyridylsulfinyl, thienylsulfinyl),
(44) an amino group,
(45) a mono- or di-$C_{1-6}$ alkylamino group (e.g., methylamino, ethylamino, propylamino, isopropylamino, butylamino, dimethylamino, diethylamino, dipropylamino, dibutylamino, N-ethyl-N-methylamino),
(46) a mono- or di-$C_{6-14}$ arylamino group (e.g., phenylamino),
(47) a 5- to 14-membered aromatic heterocyclylamino group (e.g., pyridylamino),
(48) a $C_{7-16}$ aralkylamino group (e.g., benzylamino),

(49) a formylamino group,
(50) a $C_{1-6}$ alkyl-carbonylamino group (e.g., acetylamino, propanoylamino, butanoylamino),
(51) a ($C_{1-6}$ alkyl) ($C_{1-6}$ alkyl-carbonyl)amino group (e.g., N-acetyl-N-methylamino),
(52) a $C_{6-14}$ aryl-carbonylamino group (e.g., phenylcarbonylamino, naphthylcarbonylamino),
(53) a $C_{1-6}$ alkoxy-carbonylamino group (e.g., methoxycarbonylamino, ethoxycarbonylamino, propoxycarbonylamino, butoxycarbonylamino, tert-butoxycarbonylamino),
(54) a $C_{7-16}$ aralkyloxy-carbonylamino group (e.g., benzyloxycarbonylamino),
(55) a $C_{1-6}$ alkylsulfonylamino group (e.g., methylsulfonylamino, ethylsulfonylamino),
(56) a $C_{6-14}$ arylsulfonylamino group optionally substituted by a $C_{1-6}$ alkyl group (e.g., phenylsulfonylamino, toluenesulfonylamino),
(57) an optionally halogenated $C_{1-6}$ alkyl group,
(58) a $C_{2-6}$ alkenyl group,
(59) a $C_{2-6}$ alkynyl group,
(60) a $C_{3-10}$ cycloalkyl group,
(61) a $C_{3-10}$ cycloalkenyl group and
(62) a $C_{6-14}$ aryl group.

The number of the above-mentioned substituents in the "optionally substituted hydrocarbon group" is, for example, 1 to 5, preferably 1 to 3. When the number of the substituents is two or more, the respective substituents may be the same or different.

In the present specification, examples of the "heterocyclic group" (including "heterocyclic group" of "optionally substituted heterocyclic group") include (i) an aromatic heterocyclic group, (ii) a non-aromatic heterocyclic group and (iii) a 7- to 10-membered bridged heterocyclic group, each containing, as a ring-constituting atom besides carbon atom, 1 to 4 hetero atoms selected from a nitrogen atom, a sulfur atom and an oxygen atom.

In the present specification, examples of the "aromatic heterocyclic group" (including "5- to 14-membered aromatic heterocyclic group") include a 5- to 14-membered (preferably 5- to 10-membered) aromatic heterocyclic group containing, as a ring-constituting atom besides carbon atom, 1 to 4 hetero atoms selected from a nitrogen atom, a sulfur atom and an oxygen atom.

Preferable examples of the "aromatic heterocyclic group" include 5- or 6-membered monocyclic aromatic heterocyclic groups such as thienyl, furyl, pyrrolyl, imidazolyl, pyrazolyl, thiazolyl, isothiazolyl, oxazolyl, isoxazolyl, pyridyl, pyrazinyl, pyrimidinyl, pyridazinyl, 1,2,4-oxadiazolyl, 1,3,4-oxadiazolyl, 1,2,4-thiadiazolyl, 1,3,4-thiadiazolyl, triazolyl, tetrazolyl, triazinyl and the like; and 8- to 14-membered fused polycyclic (preferably bi or tricyclic) aromatic heterocyclic groups such as benzothiophenyl, benzofuranyl, benzimidazolyl, benzoxazolyl, benzisoxazolyl, benzothiazolyl, benzisothiazolyl, benzotriazolyl, imidazopyridinyl, thienopyridinyl, furopyridinyl, pyrrolopyridinyl, pyrazolopyridinyl, oxazolopyridinyl, thiazolopyridinyl, imidazopyrazinyl, imidazopyrimidinyl, thienopyrimidinyl, furopyrimidinyl, pyrrolopyrimidinyl, pyrazolopyrimidinyl, oxazolopyrimidinyl, thiazolopyrimidinyl, pyrazolotriazinyl, naphtho[2,3-b]thienyl, phenoxathiinyl, indolyl, isoindolyl, 1H-indazolyl, purinyl, isoquinolyl, quinolyl, phthalazinyl, naphthyridinyl, quinoxalinyl, quinazolinyl, cinnolinyl, carbazolyl, β-carbolinyl, phenanthridinyl, acridinyl, phenazinyl, phenothiazinyl, phenoxazinyl and the like.

In the present specification, examples of the "non-aromatic heterocyclic group" (including "3- to 14-membered non-aromatic heterocyclic group") include a 3-to 14-membered (preferably 4- to 10-membered) non-aromatic heterocyclic group containing, as a ring-constituting atom besides carbon atom, 1 to 4 hetero atoms selected from a nitrogen atom, a sulfur atom and an oxygen atom.

Preferable examples of the "non-aromatic heterocyclic group" include 3- to 8-membered monocyclic non-aromatic heterocyclic groups such as aziridinyl, oxiranyl, thiiranyl, azetidinyl, oxetanyl, thietanyl, tetrahydrothienyl, tetrahydrofuranyl, pyrrolinyl, pyrrolidinyl, imidazolinyl, imidazolidinyl, oxazolinyl, oxazolidinyl, pyrazolinyl, pyrazolidinyl, thiazolinyl, thiazolidinyl, tetrahydroisothiazolyl, tetrahydrooxazolyl, tetrahydroisooxazolyl, piperidinyl, piperazinyl, tetrahydropyridinyl, dihydropyridinyl, dihydrothiopyranyl, tetrahydropyrimidinyl, tetrahydropyridazinyl, dihydropyranyl, tetrahydropyranyl, tetrahydrothiopyranyl, morpholinyl, thiomorpholinyl, azepanyl, diazepanyl, azepinyl, oxepanyl, azocanyl, diazocanyl and the like; and

9- to 14-membered fused polycyclic (preferably bi or tricyclic) non-aromatic heterocyclic groups such as dihydrobenzofuranyl, dihydrobenzimidazolyl, dihydrobenzoxazolyl, dihydrobenzothiazolyl, dihydrobenzisothiazolyl, dihydronaphtho[2,3-b]thienyl, tetrahydroisoquinolyl, tetrahydroquinolyl, 4H-quinolizinyl, indolinyl, isoindolinyl, tetrahydrothieno[2,3-c]pyridinyl, tetrahydrobenzazepinyl, tetrahydroquinoxalinyl, tetrahydrophenanthridinyl, hexahydrophenothiazinyl, hexahydrophenoxazinyl, tetrahydrophthalazinyl, tetrahydronaphthyridinyl, tetrahydroquinazolinyl, tetrahydrocinnolinyl, tetrahydrocarbazolyl, tetrahydro-β-carbolinyl, tetrahydroacrydinyl, tetrahydrophenazinyl, tetrahydrothioxanthenyl, octahydroisoquinolyl and the like.

In the present specification, preferable examples of the "7- to 10-membered bridged heterocyclic group" include quinuclidinyl and 7-azabicyclo[2.2.1]heptanyl.

In the present specification, examples of the "nitrogen-containing heterocyclic group" include a "heterocyclic group" containing at least one nitrogen atom as a ring-constituting atom.

In the present specification, examples of the "optionally substituted heterocyclic group" include a heterocyclic group optionally having substituent(s) selected from the aforementioned substituent group A.

The number of the substituents in the "optionally substituted heterocyclic group" is, for example, 1 to 3. When the number of the substituents is two or more, the respective substituents may be the same or different.

In the present specification, examples of the "acyl group" include a formyl group, a carboxy group, a carbamoyl group, a thiocarbamoyl group, a sulfino group, a sulfo group, a sulfamoyl group and a phosphono group, each optionally having "1 or 2 substituents selected from a $C_{1-6}$ alkyl group, a $C_{2-6}$ alkenyl group, a $C_{3-10}$ cycloalkyl group, a $C_{3-10}$ cycloalkenyl group, a $C_{6-14}$ aryl group, a $C_{7-16}$ aralkyl group, a 5- to 14-membered aromatic heterocyclic group and a 3- to 14-membered non-aromatic heterocyclic group, each of which optionally has 1 to 3 substituents selected from a halogen atom, an optionally halogenated $C_{1-6}$ alkoxy group, a hydroxy group, a nitro group, a cyano group, an amino group and a carbamoyl group".

Examples of the "acyl group" also include a hydrocarbon-sulfonyl group, a heterocyclylsulfonyl group, a hydrocarbon-sulfinyl group and a heterocyclylsulfinyl group.

Here, the hydrocarbon-sulfonyl group means a hydrocarbon group-bonded sulfonyl group, the heterocyclylsulfonyl group means a heterocyclic group-bonded sulfonyl group, the hydrocarbon-sulfinyl group means a hydrocarbon group-bonded sulfinyl group and the heterocyclylsulfinyl group means a heterocyclic group-bonded sulfinyl group.

Preferable examples of the "acyl group" include a formyl group, a carboxy group, a $C_{1-6}$ alkyl-carbonyl group, a $C_{2-6}$ alkenyl-carbonyl group (e.g., crotonoyl), a $C_{3-10}$ cycloalkyl-carbonyl group (e.g., cyclobutanecarbonyl, cyclopentanecarbonyl, cyclohexanecarbonyl, cycloheptanecarbonyl), a $C_{3-10}$ cycloalkenyl-carbonyl group (e.g., 2-cyclohexenecarbonyl), a $C_{6-14}$ aryl-carbonyl group, a $C_{7-16}$ aralkyl-carbonyl group, a 5- to 14-membered aromatic heterocyclylcarbonyl group, a 3- to 14-membered non-aromatic heterocyclylcarbonyl group, a $C_{1-6}$ alkoxy-carbonyl group, a $C_{6-14}$ aryloxy-carbonyl group (e.g., phenyloxycarbonyl, naphthyloxycarbonyl), a $C_{7-16}$ aralkyloxy-carbonyl group (e.g., benzyloxycarbonyl, phenethyloxycarbonyl), a carbamoyl group, a mono- or di-$C_{1-6}$ alkyl-carbamoyl group, a mono- or di-$C_{2-6}$ alkenyl-carbamoyl group (e.g., diallylcarbamoyl), a mono- or di-$C_{3-10}$ cycloalkyl-carbamoyl group (e.g., cyclopropylcarbamoyl), a mono- or di-$C_{6-14}$ aryl-carbamoyl group (e.g., phenylcarbamoyl), a mono- or di-$C_{7-16}$ aralkyl-carbamoyl group, a 5- to 14-membered aromatic heterocyclylcarbamoyl group (e.g., pyridylcarbamoyl), a thiocarbamoyl group, a mono- or di-$C_{1-6}$ alkyl-thiocarbamoyl group (e.g., methylthiocarbamoyl, N-ethyl-N-methylthiocarbamoyl), a mono- or di-$C_{2-6}$ alkenyl-thiocarbamoyl group (e.g., diallylthiocarbamoyl), a mono- or di-$C_{3-10}$ cycloalkyl-thiocarbamoyl group (e.g., cyclopropylthiocarbamoyl, cyclohexylthiocarbamoyl), a mono- or di-$C_{6-14}$ aryl-thiocarbamoyl group (e.g., phenylthiocarbamoyl), a mono- or di-$C_{7-16}$ aralkyl-thiocarbamoyl group (e.g., benzylthiocarbamoyl, phenethylthiocarbamoyl), a 5- to 14-membered aromatic heterocyclylthiocarbamoyl group (e.g., pyridylthiocarbamoyl), a sulfino group, a $C_{1-6}$ alkylsulfinyl group (e.g., methylsulfinyl, ethylsulfinyl), a sulfo group, a $C_{1-6}$ alkylsulfonyl group, a $C_{6-14}$ arylsulfonyl group, a phosphono group and a mono- or di-$C_{1-6}$ alkylphosphono group (e.g., dimethylphosphono, diethylphosphono, diisopropylphosphono, dibutylphosphono).

In the present specification, examples of the "optionally substituted amino group" include an amino group optionally having "1 or 2 substituents selected from a $C_{1-6}$ alkyl group, a $C_{2-6}$ alkenyl group, a $C_{3-10}$ cycloalkyl group, a $C_{6-14}$ aryl group, a $C_{7-16}$ aralkyl group, a $C_{1-6}$ alkyl-carbonyl group, a $C_{6-14}$ aryl-carbonyl group, a $C_{7-16}$ aralkyl-carbonyl group, a 5- to 14-membered aromatic heterocyclylcarbonyl group, a 3- to 14-membered non-aromatic heterocyclylcarbonyl group, a $C_{1-6}$ alkoxy-carbonyl group, a 5- to 14-membered aromatic heterocyclic group, a carbamoyl group, a mono- or di-$C_{1-6}$ alkyl-carbamoyl group, a mono- or di-$C_{7-16}$ aralkyl-carbamoyl group, a $C_{1-6}$ alkylsulfonyl group and a $C_{6-14}$ arylsulfonyl group, each of which optionally has 1 to 3 substituents selected from substituent group A".

Preferable examples of the optionally substituted amino group include an amino group, a mono- or di-(optionally halogenated $C_{1-6}$ alkyl)amino group (e.g., methylamino, trifluoromethylamino, dimethylamino, ethylamino, diethylamino, propylamino, dibutylamino), a mono- or di-$C_{2-6}$ alkenylamino group (e.g., diallylamino), a mono- or di-$C_{3-10}$ cycloalkylamino group (e.g., cyclopropylamino, cyclohexylamino), a mono- or di-$C_{6-14}$ arylamino group (e.g., phenylamino), a mono- or di-$C_{7-16}$ aralkylamino group (e.g., benzylamino, dibenzylamino), a mono- or di-(optionally halogenated $C_{1-6}$ alkyl)-carbonylamino group (e.g., acetylamino, propionylamino), a mono- or di-$C_{6-14}$ aryl-carbonylamino group (e.g., benzoylamino), a mono- or di-$C_{7-16}$ aralkyl-carbonylamino group (e.g., benzylcarbonylamino), a mono- or di-5- to 14-membered aromatic heterocyclylcarbonylamino group (e.g., nicotinoylamino, isonicotinoylamino), a mono- or di-3- to 14-membered non-aromatic heterocyclylcarbonylamino group (e.g., piperidinylcarbonylamino), a mono- or di-$C_{1-6}$ alkoxy-carbonylamino group (e.g., tert-butoxycarbonylamino), a 5- to 14-membered aromatic heterocyclylamino group (e.g., pyridylamino), a carbamoylamino group, a (mono- or di-$C_{1-6}$ alkyl-carbamoyl)amino group (e.g., methylcarbamoylamino), a (mono- or di-$C_{7-16}$ aralkyl-carbamoyl) amino group (e.g., benzylcarbamoylamino), a $C_{1-6}$ alkylsulfonylamino group (e.g., methylsulfonylamino, ethylsulfonylamino), a $C_{6-14}$ arylsulfonylamino group (e.g., phenylsulfonylamino), a ($C_{1-6}$ alkyl) ($C_{1-6}$ alkyl-carbonyl)amino group (e.g., N-acetyl-N-methylamino) and a ($C_{1-6}$ alkyl) ($C_{6-14}$ aryl-carbonyl)amino group (e.g., N-benzoyl-N-methylamino).

In the present specification, examples of the "optionally substituted carbamoyl group" include a carbamoyl group optionally having "1 or 2 substituents selected from a $C_{1-6}$ alkyl group, a $C_{2-6}$ alkenyl group, a $C_{3-10}$ cycloalkyl group, a $C_{6-14}$ aryl group, a $C_{7-16}$ aralkyl group, a $C_{1-6}$ alkyl-carbonyl group, a $C_{6-14}$ aryl-carbonyl group, a $C_{7-16}$ aralkyl-carbonyl group, a 5- to 14-membered aromatic heterocyclylcarbonyl group, a 3- to 14-membered non-aromatic heterocyclylcarbonyl group, a $C_{1-6}$ alkoxy-carbonyl group, a 5- to 14-membered aromatic heterocyclic group, a carbamoyl group, a mono- or di-$C_{1-6}$ alkyl-carbamoyl group and a mono- or di-$C_{7-16}$ aralkyl-carbamoyl group, each of which optionally has 1 to 3 substituents selected from substituent group A".

Preferable examples of the optionally substituted carbamoyl group include a carbamoyl group, a mono- or di-$C_{1-6}$ alkyl-carbamoyl group, a mono- or di-$C_{2-6}$ alkenyl-carbamoyl group (e.g., diallylcarbamoyl), a mono- or di-$C_{3-10}$ cycloalkyl-carbamoyl group (e.g., cyclopropylcarbamoyl, cyclohexylcarbamoyl), a mono- or di-$C_{6-14}$ aryl-carbamoyl group (e.g., phenylcarbamoyl), a mono- or di-$C_{7-16}$ aralkyl-carbamoyl group, a mono- or di-$C_{1-6}$ alkyl-carbonyl-carbamoyl group (e.g., acetylcarbamoyl, propionylcarbamoyl), a mono- or di-$C_{6-14}$ aryl-carbonyl-carbamoyl group (e.g., benzoylcarbamoyl) and a 5- to 14-membered aromatic heterocyclylcarbamoyl group (e.g., pyridylcarbamoyl).

In the present specification, examples of the "optionally substituted thiocarbamoyl group" include a thiocarbamoyl group optionally having "1 or 2 substituents selected from a $C_{1-6}$ alkyl group, a $C_{2-6}$ alkenyl group, a $C_{3-10}$ cycloalkyl group, a $C_{6-14}$ aryl group, a $C_{7-16}$ aralkyl group, a $C_{1-6}$ alkyl-carbonyl group, a $C_{6-14}$ aryl-carbonyl group, a $C_{7-16}$ aralkyl-carbonyl group, a 5-to 14-membered aromatic heterocyclylcarbonyl group, a 3-to 14-membered non-aromatic heterocyclylcarbonyl group, a $C_{1-6}$ alkoxy-carbonyl group, a 5- to 14-membered aromatic heterocyclic group, a carbamoyl group, a mono- or di-$C_{1-6}$ alkyl-carbamoyl group and a mono- or di-$C_{7-16}$ aralkyl-carbamoyl group, each of which optionally has 1 to 3 substituents selected from substituent group A".

Preferable examples of the optionally substituted thiocarbamoyl group include a thiocarbamoyl group, a mono- or di-$C_{1-6}$ alkyl-thiocarbamoyl group (e.g., methylthiocarbamoyl, ethylthiocarbamoyl, dimethylthiocarbamoyl, diethylthiocarbamoyl, N-ethyl-N-methylthiocarbamoyl), a mono- or di-$C_{2-6}$ alkenyl-thiocarbamoyl group (e.g., diallylthiocarbamoyl), a mono- or di-$C_{3-10}$ cycloalkyl-thiocarbamoyl group (e.g., cyclopropylthiocarbamoyl, cyclohexylthiocarbamoyl), a mono- or di-$C_{6-14}$ aryl-thiocarbamoyl group (e.g., phenylthiocarbamoyl), a mono- or di-$C_{7-16}$ aralkyl-thiocarbamoyl group (e.g., benzylthiocarbamoyl, phenethylthiocarbamoyl), a mono- or di-$C_{1-6}$ alkyl-carbonyl-thiocarbamoyl group (e.g., acetylthiocarbamoyl, propionylthiocarbamoyl), a mono- or di-$C_{6-14}$ aryl-carbonyl-thiocarbamoyl group (e.g., benzoylthiocarbamoyl) and a 5- to 14-membered aromatic heterocyclylthiocarbamoyl group (e.g., pyridylthiocarbamoyl).

In the present specification, examples of the "optionally substituted sulfamoyl group" include a sulfamoyl group optionally having "1 or 2 substituents selected from a $C_{1-6}$ alkyl group, a $C_{2-6}$ alkenyl group, a $C_{3-10}$ cycloalkyl group, a $C_{6-14}$ aryl group, a $C_{7-16}$ aralkyl group, a $C_{1-6}$ alkyl-carbonyl group, a $C_{6-14}$ aryl-carbonyl group, a $C_{7-16}$ aralkyl-carbonyl group, a 5- to 14-membered aromatic heterocyclylcarbonyl group, a 3- to 14-membered non-aromatic heterocyclylcarbonyl group, a $C_{1-6}$ alkoxy-carbonyl group, a 5- to 14-membered aromatic heterocyclic group, a carbamoyl group, a mono- or di-$C_{1-6}$ alkyl-carbamoyl group and a mono- or di-$C_{7-16}$ aralkyl-carbamoyl group, each of which optionally has 1 to 3 substituents selected from substituent group A".

Preferable examples of the optionally substituted sulfamoyl group include a sulfamoyl group, a mono- or di-$C_{1-6}$ alkyl-sulfamoyl group (e.g., methylsulfamoyl, ethylsulfamoyl, dimethylsulfamoyl, diethylsulfamoyl, N-ethyl-N-methylsulfamoyl), a mono- or di-$C_{2-6}$ alkenyl-sulfamoyl group (e.g., diallylsulfamoyl), a mono- or di-$C_{3-10}$ cycloalkyl-sulfamoyl group (e.g., cyclopropylsulfamoyl, cyclohexylsulfamoyl), a mono- or di-$C_{6-14}$ aryl-sulfamoyl group (e.g., phenylsulfamoyl), a mono- or di-$C_{7-16}$ aralkyl-sulfamoyl group (e.g., benzylsulfamoyl, phenethylsulfamoyl), a mono- or di-$C_{1-6}$ alkyl-carbonyl-sulfamoyl group (e.g., acetylsulfamoyl, propionylsulfamoyl), a mono- or di-$C_{6-14}$ aryl-carbonyl-sulfamoyl group (e.g., benzoylsulfamoyl) and a 5- to 14-membered aromatic heterocyclylsulfamoyl group (e.g., pyridylsulfamoyl).

In the present specification, examples of the "optionally substituted hydroxy group" include a hydroxyl group optionally having "a substituent selected from a $C_{1-6}$ alkyl group, a $C_{2-6}$ alkenyl group, a $C_{3-10}$ cycloalkyl group, a $C_{6-14}$ aryl group, a $C_{7-16}$ aralkyl group, a $C_{1-6}$ alkyl-carbonyl group, a $C_{6-14}$ aryl-carbonyl group, a $C_{7-16}$ aralkyl-carbonyl group, a 5- to 14-membered aromatic heterocyclylcarbonyl group, a 3- to 14-membered non-aromatic heterocyclylcarbonyl group, a $C_{1-6}$ alkoxy-carbonyl group, a 5- to 14-membered aromatic heterocyclic group, a carbamoyl group, a mono- or di-$C_{1-6}$ alkyl-carbamoyl group, a mono- or di-$C_{7-16}$ aralkyl-carbamoyl group, a $C_{1-6}$ alkylsulfonyl group and a $C_{6-14}$ arylsulfonyl group, each of which optionally has 1 to 3 substituents selected from substituent group A".

Preferable examples of the optionally substituted hydroxy group include a hydroxy group, a $C_{1-6}$ alkoxy group, a $C_{2-6}$ alkenyloxy group (e.g., allyloxy, 2-butenyloxy, 2-pentenyloxy, 3-hexenyloxy), a $C_{3-10}$ cycloalkyloxy group (e.g., cyclohexyloxy), a $C_{6-14}$ aryloxy group (e.g., phenoxy, naphthyloxy), a $C_{7-16}$ aralkyloxy group (e.g., benzyloxy, phenethyloxy), a $C_{1-6}$ alkyl-carbonyloxy group (e.g., acetyloxy, propionyloxy, butyryloxy, isobutyryloxy, pivaloyloxy), a $C_{6-14}$ aryl-carbonyloxy group (e.g., benzoyloxy), a $C_{7-16}$ aralkyl-carbonyloxy group (e.g., benzylcarbonyloxy), a 5- to 14-membered aromatic heterocyclylcarbonyloxy group (e.g., nicotinoyloxy), a 3-to 14-membered non-aromatic heterocyclylcarbonyloxy group (e.g., piperidinylcarbonyloxy), a $C_{1-6}$ alkoxy-carbonyloxy group (e.g., tert-butoxycarbonyloxy), a 5- to 14-membered aromatic heterocyclyloxy group (e.g., pyridyloxy), a carbamoyloxy group, a $C_{1-6}$ alkyl-carbamoyloxy group (e.g., methylcarbamoyloxy), a $C_{7-16}$ aralkyl-carbamoyloxy group (e.g., benzylcarbamoyloxy), a $C_{1-6}$ alkylsulfonyloxy group (e.g.,

methylsulfonyloxy, ethylsulfonyloxy) and a $C_{6-14}$ arylsulfonyloxy group (e.g., phenylsulfonyloxy).

In the present specification, examples of the "optionally substituted sulfanyl group" include a sulfanyl group optionally having "a substituent selected from a $C_{1-6}$ alkyl group, a $C_{2-6}$ alkenyl group, a $C_{3-10}$ cycloalkyl group, a $C_{6-14}$ aryl group, a $C_{7-16}$ aralkyl group, a $C_{1-6}$ alkyl-carbonyl group, a $C_{6-14}$ aryl-carbonyl group and a 5- to 14-membered aromatic heterocyclic group, each of which optionally has 1 to 3 substituents selected from substituent group A" and a halogenated sulfanyl group.

Preferable examples of the optionally substituted sulfanyl group include a sulfanyl (-SH) group, a $C_{1-6}$ alkylthio group, a $C_{2-6}$ alkenylthio group (e.g., allylthio, 2-butenylthio, 2-pentenylthio, 3-hexenylthio), a $C_{3-10}$ cycloalkylthio group (e.g., cyclohexylthio), a $C_{6-14}$ arylthio group (e.g., phenylthio, naphthylthio), a $C_{7-16}$ aralkylthio group (e.g., benzylthio, phenethylthio), a $C_{1-6}$ alkyl-carbonylthio group (e.g., acetylthio, propionylthio, butyrylthio, isobutyrylthio, pivaloylthio), a $C_{6-14}$ aryl-carbonylthio group (e.g., benzoylthio), a 5-to 14-membered aromatic heterocyclylthio group (e.g., pyridylthio) and a halogenated thio group (e.g., pentafluorothio).

In the present specification, examples of the "optionally substituted silyl group" include a silyl group optionally having "1 to 3 substituents selected from a $C_{1-6}$ alkyl group, a $C_{2-6}$ alkenyl group, a $C_{3-10}$ cycloalkyl group, a $C_{6-14}$ aryl group and a $C_{7-16}$ aralkyl group, each of which optionally has 1 to 3 substituents selected from substituent group A".

Preferable examples of the optionally substituted silyl group include a tri-$C_{1-6}$ alkylsilyl group (e.g., trimethylsilyl, tert-butyl(dimethyl)silyl).

Available "factor having CDK8/19-inhibiting activity" includes, for example, compounds 1 to 7 described below and in Figure 1 or salts thereof. Compounds 1 to 5 are preferably in a free form, and compounds 6 and 7 are preferably in a trifluoroacetate form.

[Formula 2]

| Compound | IUPAC name | Structural formula | Salt | **MS** |
|---|---|---|---|---|
| 1 | 8-(2,4-Difluorophenoxy)-1-methyl-4,5-dihydro-1 H-thieno[3,4-g]indazole-6-carboxamide | 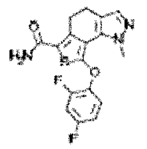 | | |
| 2 | 4-((4-Fluorophenyl)sulfonyl)-3-(2-(imidazo[1,2-b] pyridazin-6-ylsulfanyl)ethyl)-3,4-dihydroquinoxalin-2 (1H)-one | 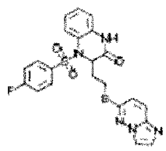 | | |
| 3 | 2-(Benzylamino)-4-(1H-pyrrolo[2,3-b]pyridin-3-yl) benzamide | 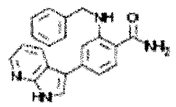 | | 343.2 |
| 4 | 3-(3-(Benzyloxy)phenyl)-1H-pyrrolo[2,3-b]pyridine | 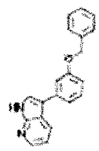 | | |
| 5 | 4-(4-(2,3-Dihydro-1,4-benzodioxin-6-yl)-1H-pyrazol-3-yl)benzene-1,3-diol | 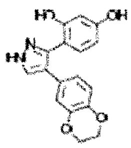 | | |
| 6 | N-Butyl-8-(4-methoxyphenyl)-1,6-naphthyridine-2-carboxamide | 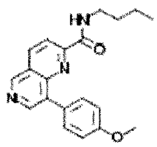 | $CF_3COOH$ | |

(continued)

| Compound | IUPAC name | Structural formula | Salt | **MS** |
|---|---|---|---|---|
| 7 | 8-(4-Methylphenyl)-N,N-dipropyl-1,6-naphthyridine-2-carboxamide | 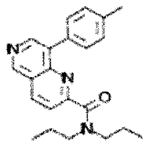 | $CF_3COOH$ | |

The factor having CDK8/19-inhibiting activity is preferably allowed to act on cells in the presence of a growth factor. The growth factor is preferably EGF, KGF, and/or FGF10, more preferably EGF and/or KGF, further preferably EGF and KGF.

The factor having CDK8/19-inhibiting activity enriches PDX-1-positive cells at the same time with NKX6.1-positive cells. Specifically, the factor having CDK8/19-inhibiting activity enriches NKX6.1-positive and PDX-1-positive cells.

3. Method for producing pancreatic progenitor cells (induction of differentiation)

The process of inducing pancreatic progenitor cells from pluripotent stem cells can be divided into four stages: stage 1: the differentiation of the pluripotent stem cells into definitive endoderm cells, stage 2: the differentiation of the definitive endoderm cells into primitive gut tube cells, stage 3: the differentiation of the primitive gut tube cells into posterior foregut cells, and stage 4: the differentiation of the posterior foregut cells into pancreatic progenitor cells. The factor having CDK8/19-inhibiting activity acts on the posterior foregut cell population, particularly, at stage 4, to promote its differentiation into pancreatic progenitor cells.

The present invention also provides a method for producing NKX6.1-positive and PDX-1-positive pancreatic progenitor cells from pluripotent stem cells using a factor having CDK8/19-inhibiting activity. The method comprises the following steps:

step 1) inducing the differentiation of human pluripotent stem cells into definitive endoderm cells;
step 2) inducing the differentiation of the definitive endoderm cells into primitive gut tube cells;
step 3) inducing the differentiation of the primitive gut tube cells into posterior foregut cells; and
step 4) culturing the posterior foregut cells in a medium containing the factor having CDK8/19-inhibiting activity.

The pluripotent stem cells are cultured in a medium for pluripotent stem cells according to a routine method prior to differentiation culture. Commercially available StemFit(R) (ReproCELL Inc.) or the like can be used as the medium for pluripotent stem cells. In this operation, the cells may be cultured for 1 day or overnight in advance, if necessary, in a medium containing a ROCK inhibitor (for example, Y-27632) or the like.

Step 1) Differentiation into definitive endoderm

The human pluripotent stem cells are first allowed to differentiate into definitive endoderm cells. Methods for inducing the definitive endoderm from human pluripotent stem cells has already been known, and any of the methods may be used. Preferably, the human pluripotent stem cells are cultured in a medium containing activin A, more preferably a medium containing activin A, a ROCK inhibitor, and a GSK3β inhibitor, to thereby differentiate into definitive endoderm cells. The number of cells at the start of culture is not particularly limited and is 22000 to 150000 cells/$cm^2$, preferably 22000 to 100000 cells/$cm^2$, more preferably 22000 to 80000 cells/$cm^2$. The culture period is 1 day to 4 days, preferably 1 day to 3 days, particularly preferably 3 days.

The culture temperature is not particularly limited, and the culture is performed at 30 to 40°C (for example, 37°C). The concentration of carbon dioxide in a culture container is on the order of, for example, 5%.

The medium used in this step may be a basal medium for use in the culture of mammalian cells, such as RPMI 1640 medium, MEM medium, iMEM medium, DMEM/F12 medium, or Improved MEM Zinc Option medium.

The concentration of the activin A in the medium is usually 30 to 200 ng/mL, preferably 50 to 150 ng/mL, more preferably 70 to 120 ng/mL, particularly preferably about 100 ng/mL.

The concentration of the GSK3β inhibitor in the medium is appropriately set depending on the type of the GSK3β inhibitor used. For example, in the case of using CHIR as the GSK3β inhibitor, its concentration is usually 2 to 5 μM, preferably 2 to 4 μM, particularly preferably about 3 μM.

The concentration of the ROCK inhibitor in the medium is appropriately set depending on the type of the ROCK

inhibitor used. For example, in the case of using Y-27632 as the ROCK inhibitor, its concentration is usually 5 to 20 μM, preferably 5 to 15 μM, particularly preferably about 10 μM.

Specifically, the cells are cultured for 1 day in a medium containing activin A, a ROCK inhibitor, and a GSK3β inhibitor and then further cultured for 2 days in a medium containing only activin A with the medium replaced with a fresh one every day.

Step 2) Differentiation into primitive gut tube cells

The definitive endoderm cells obtained in stage 1) are further cultured in a medium containing a growth factor to induce their differentiation into primitive gut tube cells. The culture period is 2 days to 8 days, preferably about 4 days.

The culture temperature is not particularly limited, and the culture is performed at 30 to 40°C (for example, 37°C). The concentration of carbon dioxide in a culture container is on the order of, for example, 5%.

A basal medium for use in the culture of mammalian cells can be used as culture medium, as in step 1). The medium may be appropriately supplemented with a serum replacement, a vitamin, an antibiotic, and the like, in addition to the growth factor.

The growth factor is preferably EGF, KGF, and/or FGF10, more preferably EGF and/or KGF, further preferably KGF.

The concentration of the growth factor in the medium is appropriately set depending on the type of the growth factor used and is usually about 0.1 nM to 1000 μM, preferably about 0.1 nM to 100 μM. In the case of EGF, its concentration is about 5 to 2000 ng/ml (that is, about 0.8 to 320 nM), preferably about 5 to 1000 ng/ml (that is, about 0.8 to 160 nM), more preferably about 10 to 1000 ng/ml (that is, about 1.6 to 160 nM). In the case of FGF10, its concentration is about 5 to 2000 ng/ml (that is, about 0.3 to 116 nM), preferably about 10 to 1000 ng/ml (that is, about 0.6 to 58 nM), more preferably about 10 to 1000 ng/ml (that is, about 0.6 to 58 nM).
For example, in the case of using KGF as the growth factor, its concentration is usually 5 to 150 ng/mL, preferably 30 to 100 ng/mL, particularly preferably about 50 ng/mL.

Step 3) Differentiation into posterior foregut cells

The primitive gut tube cells obtained in step 2) are further cultured in a medium containing a growth factor, cyclopamine, noggin, and the like to induce their differentiation into posterior foregut cells. The culture period is 1 day to 5 days, preferably about 2 days.

The culture temperature is not particularly limited, and the culture is performed at 30 to 40°C (for example, 37°C). The concentration of carbon dioxide in a culture container is on the order of, for example, 5%.

As in step 1), a basal medium for use in the culture of mammalian cells can be used as culture medium. The medium may be appropriately supplemented with a serum replacement, a vitamin, an antibiotic, and the like, in addition to the growth factor.

The growth factor is preferably EGF, KGF, and/or FGF10, more preferably EGF and/or KGF, further preferably KGF.

The concentration of the growth factor in the medium is appropriately set depending on the type of the growth factor used and is usually about 0.1 nM to 1000 μM, preferably about 0.1 nM to 100 μM. In the case of EGF, its concentration is about 5 to 2000 ng/ml (that is, about 0.8 to 320 nM), preferably about 5 to 1000 ng/ml (that is, about 0.8 to 160 nM), more preferably about 10 to 1000 ng/ml (that is, about 1.6 to 160 nM). In the case of FGF10, its concentration is about 5 to 2000 ng/ml (that is, about 0.3 to 116 nM), preferably about 10 to 1000 ng/ml (that is, about 0.6 to 58 nM), more preferably about 10 to 1000 ng/ml (that is, about 0.6 to 58 nM).
For example, in the case of using KGF as the growth factor, its concentration is usually 5 to 150 ng/mL, preferably 30 to 100 ng/mL, particularly preferably about 50 ng/mL.

The concentration of the cyclopamine in the medium is not particularly limited and is usually 0.5 to 1.5 μM, preferably 0.3 to 1.0 μM, particularly preferably about 0.5 μM.

The concentration of the noggin in the medium is not particularly limited and is usually 10 to 200 ng/mL, preferably 50 to 150 ng/mL, particularly preferably about 100 ng/mL.

Step 4) Differentiation into pancreatic progenitor cells

The posterior foregut cells obtained in step 3) are further cultured in a medium containing a factor having CDK8/19-inhibiting activity, preferably a medium containing a factor having CDK8/19-inhibiting activity and a growth factor, to induce their differentiation into pancreatic progenitor cells (cell population comprising NKX6.1-positive and PDX1-positive cells). The culture period is 2 days to 10 days, preferably about 5 days.

According to the previous report (Toyoda et al., Stem cell Research (2015) 14, 185-197), the posterior foregut

cells obtained in step 3) are treated with 0.25% trypsin-EDTA and then dispersed by pipetting. After centrifugal separation of 0.25% trypsin-EDTA, the cells were suspended and then reseed to a fresh medium of step 4.

As in step 1), a basal medium for use in the culture of mammalian cells can be used as culture medium. The medium may be appropriately supplemented with a serum replacement, a vitamin, an antibiotic, and the like, in addition to the growth factor.

Each of the compounds mentioned above or salts thereof can be used as the factor having CDK8/19-inhibiting activity. The amount of the factor added to the medium is appropriately determined according to the compound or the salt thereof used and is usually about 0.00001 μM to 5 μM, preferably 0.00001 μM to 1 μM. The concentration of the factor having CDK8/19-inhibiting activity in the medium is preferably a concentration that attains inhibitory activity of 50% or more for CDK8/19.

The growth factor is preferably EGF, KGF, and/or FGF10, more preferably KGF and/or EGF, further preferably KGF and EGF.

The concentration of the growth factor in the medium is appropriately set depending on the type of the growth factor used and is usually about 0.1 nM to 1000 μM, preferably about 0.1 nM to 100 μM. In the case of EGF, its concentration is about 5 to 2000 ng/ml (that is, about 0.8 to 320 nM), preferably about 5 to 1000 ng/ml (that is, about 0.8 to 160 nM), more preferably about 10 to 1000 ng/ml (that is, about 1.6 to 160 nM). In the case of FGF10, its concentration is about 5 to 2000 ng/ml (that is, about 0.3 to 116 nM), preferably about 10 to 1000 ng/ml (that is, about 0.6 to 58 nM), more preferably about 10 to 1000 ng/ml (that is, about 0.6 to 58 nM).
For example, in the case of using KGF and EGF as the growth factor, the concentration of KGF is usually 5 to 150 ng/mL, preferably 30 to 100 ng/mL, particularly preferably about 50 ng/mL, and the concentration of EGF is usually 10 to 200 ng/mL, preferably 50 to 150 ng/mL, particularly preferably about 100 ng/mL.

Culture on the first day in step 4) may be performed in the presence of a ROCK inhibitor, and culture on the following days may be performed in a medium containing no ROCK inhibitor.

The action of the factor having CDK8/19-inhibiting activity has been confirmed to be effective for the induction of differentiation even after aggregate formation.

In any of the steps, the medium may be supplemented with a serum replacement (for example, B-27 supplement, ITS-G), in addition to the components described above. Also, an amino acid, L-glutamine, GlutaMAX (product name), a non-essential amino acid, a vitamin, an antibiotic (for example, Antibiotic-Antimycotic (also referred to as AA herein), penicillin, streptomycin, or a mixture thereof), an antimicrobial agent (for example, amphotericin B), an antioxidant, pyruvic acid, a buffer, inorganic salts, and the like may be added thereto, if necessary. In the case of adding an antibiotic to the medium, its concentration in the medium is usually 0.01 to 20% by weight, preferably 0.1 to 10% by weight.

The cell culture is performed by adherent culture without the use of feeder cells. For the culture, a culture container, for example, a dish, a flask, a microplate, or a cell culture sheet such as OptiCell (product name) (Nunc), is used. The culture container is preferably surface-treated in order to improve adhesiveness to cells (hydrophilicity), or coated with a substrate for cell adhesion such as collagen, gelatin, poly-L-lysine, poly-D-lysine, laminin, fibronectin, Matrigel (for example, BD Matrigel (Nippon Becton Dickinson Company, Ltd.)), or vitronectin. The culture container is preferably a culture container coated with type I-collagen, Matrigel, fibronectin, vitronectin or poly-D-lysine, more preferably a culture container coated with Matrigel or poly-D-lysine.

The culture temperature is not particularly limited, and the culture is performed at 30 to 40°C (for example, 37°C). The concentration of carbon dioxide in a culture container is on the order of, for example, 5%.

The pancreatic progenitor cells obtained in step 4) can be further purified using a surface marker PDX1 and/or NKX6.1. The purification can be performed by a method known per se, for example, using anti-PDX-1 antibody- and/or anti-NKX6.1 antibody-immobilized beads.

4. Culture enriched for NKX6.1-positive and PDX-1-positive cells

The present invention also provides a cell culture enriched for NKX6.1-positive and PDX-1-positive cells, the cell culture comprising human cells and a factor having CDK8/19-inhibiting activity. In the cell culture, at least about 10% of the human cells are NKX6.1-positive and PDX-1-positive cells, and preferably about 30%, more preferably about 60%, further preferably about 70%, particularly preferably about 80%, most preferably about 90%, of the human cells are NKX6.1-positive and PDX-1-positive cells.

5. NKX6.1-positive and PDX-1-positive cells

The present invention also provides human pluripotent stem cell-derived NKX6.1-positive and PDX-1-positive cells produced using a factor having CDK8/19-inhibiting activity.

The present invention also provides a medicament comprising human pluripotent stem cell-derived NKX6.1-positive and PDX-1-positive cells produced using a factor having CDK8/19-inhibiting activity. The NKX6.1-positive and

PDX-1-positive cells function as pancreatic progenitor cells. The NKX6.1-positive and PDX-1-positive cells differentiate into β cells which may produce insulin according to a method described in a known literature, and are thereby useful in the treatment of diabetes mellitus or the like ascribable to β cell failure. Hence, the medicament of the present invention is transplanted as it is or in a capsule form to an affected area and is thereby useful as a cell medicine or device for treating diabetes mellitus, particularly, type I diabetes mellitus.

6. Inducer of differentiation into pancreatic progenitor cells

NKX6.1-positive and PDX-1-positive cells induced by a factor having CDK8/19-inhibiting activity function as pancreatic progenitor cells. Accordingly, the factor having CDK8/19-inhibiting activity can be utilized as an inducer of differentiation into pancreatic progenitor cells. Also, a medium containing the factor having CDK8/19-inhibiting activity can be utilized as a medium for induction of differentiation into pancreatic progenitor cells.

As used herein, use of a medium containing a factor having CDK8/19-inhibiting activity as a medium for induction of differentiation into pancreatic progenitor cells is carried out *in vitro* or/and *in vivo.*

Examples

Hereinafter, the present invention will be specifically described with reference to Reference Example and Examples. However, the present invention is not limited by these examples.

Reference Example 1: Maintenance culture of Ff-WJ-18 cells as human iPS cells

The human iPS cells used were Ff-WJ-18 cells (Kyoto University).

The maintenance culture of the Ff-WJ-18 cells employed StemFit(R) AK03N medium (Ajinomoto Healthy Supply Co., Inc.) as a medium, and a culture container coated with iMatrix-511 solution (Wako Pure Chemical Industries, Ltd.). The cells were cultured at 37°C under 5% $CO_2$.

In addition to the Ff-WJ-18 cells, Ff-I01-S1 cells (Kyoto University) were also subjected as human iPS cells to a similar test.

Example 1: Evaluation of CDK8/19 inhibitor for its CDK8-inhibiting activity and CDK19-inhibiting activity

1. Synthesis of CDK8/19 inhibitor (see Figure 1)

(1) Compound 1

8-(2,4-Difluorophenoxy)-1-methyl-4,5-dihydro-1H-thieno[3,4-g]indazole-6-carboxamide

Compound 1 or a salt thereof is produced by the method described in WO2001/074823 or a method equivalent thereto.

(2) Compound 2

4-((4-Fluorophenyl)sulfonyl)-3-(2-(imidazo[1,2-b]pyridazin-6-ylsulfanyl)ethyl)-3,4-dihydroquinoxalin-2(1H)-one

Compound 2 or a salt thereof is produced by the method described in US2005-0020591 and a known method, or a method equivalent thereto.

(3) Compound 3

2-(Benzylamino)-4-(1H-pyrrolo[2,3-b]pyridin-3-yl)benzamide

Compound 3 or a salt thereof is produced by the method described in Bioorganic & Medicinal Chemistry, 24(11), 2466-2475; 2016 or WO2005/095400 and a known method, or a method equivalent thereto.

(4) Compound 4

3-(3-(Benzyloxy)phenyl)-1H-pyrrolo[2,3-b]pyridine

Compound 4 or a salt thereof is produced by the method described in WO2005/095400 or a method equivalent thereto.

(5) Compound 5

4-(4-(2,3-Dihydro-1,4-benzodioxin-6-yl)-1H-pyrazol-3-yl)benzene-1,3-diol

Compound 5 or a salt thereof can be obtained as a commercially available product (see WO2015/159937).

(6) Compound 6

N-Butyl-8-(4-methoxyphenyl)-1,6-naphthyridine-2-carboxamide trifluoroacetate

Compound 6 or a salt thereof is produced by the method described in WO2014079787 and a known method, or a method equivalent thereto.

(7) Compound 7

8-(4-Methylphenyl)-N,N-dipropyl-1,6-naphthyridine-2-carboxamide trifluoroacetate

Compound 7 or a salt thereof is produced by the method described in WO2014/079787 and a known method, or a method equivalent thereto.

2. CDK8-inhibiting activity and CDK19-inhibiting activity

Test compounds were evaluated for their CDK8-inhibiting activity and CDK19-inhibiting activity by the following method.

Each test compound dissolved in dimethyl sulfoxide was diluted with an assay buffer (25 mM HEPES, 10 mM $MgCl_2$, 2 mM DL-dithiothreitol, 0.01 % Tween-20) to obtain a primary dilution having a DMSO concentration of 3%. The primary dilution was dispensed at 2 μL/well to a 384-well plate. Then, a mixed solution of $Eu^{3+}$ Cryptate conjugated mouse monoclonal antibody anti-glutathione S-transferase (CisBio) diluted 267-fold with an assay buffer and 60 nM Kinase Tracer-236 (Life Technologies) was added at 2 μL/well. After the addition, a kinase solution (84 ng/mL CDK8/CycC (Carna Biosciences) diluted with an assay buffer was used for CDK8-inhibiting activity measurement, and 87 ng/mL CDC2L6/CycC (Carna Biosciences) diluted with an assay buffer was used for CDK19-inhibiting activity measurement) was further added at 2 μL/well. After the addition, the plate was left standing at room temperature for 1 hour, followed by the measurement of fluorescence intensity (excitation wavelength: 320 nm, fluorescence wavelength: 615 nm, 665 nm, delay time: 50 μsec) using a plate reader EnVision (Perkin Elmer).

When the fluorescence intensity of a reaction solution under compound non-addition conditions was used as a control and the fluorescence intensity of a reaction solution under 10 μM control compound addition conditions was used as a blank, the rate of inhibition of CDK8 or CDK19 by the test compound can be calculated according to the following expression:

$$\text{Rate of inhibition (\%)} = (1- (\text{Fluorescence intensity of the test compound} - \text{Blank}) / (\text{Control} - \text{Blank})) \times 100$$

The control compound used was commercially available 4-(4-(2,3-dihydro-1,4-benzodioxin-6-yl)-1H-pyrazol-3-yl)benzene-1,3-diol. For reference, the concentration of the control compound necessary for exhibiting a 50% rate of inhibition of CDK8 or CDK19 ($IC_{50}$ value) is shown in Table 1.

[Table 1]

| CDK8 inhibition $IC_{50}$ (μM) | CDK19 inhibition $IC_{50}$ (μM) |
|---|---|
| 0.12 | 0.13 |

The rate of inhibition of CDK8 (%) and the rate of inhibition of CDK19 (%) at 10 μM and 1 μM of the test compound are shown in Table 2.

[Table 2]

| | CDK8 | | CDK19 | |
|---|---|---|---|---|
| | 10 μM | 1 μM | 10 μM | 1 μM |
| Compound 1 | 94 | 97 | 96 | 99 |
| Compound 2 | 100 | 100 | 103 | 102 |
| Compound 3 | 105 | 101 | 104 | 101 |
| Compound 4 | 102 | 91 | 101 | 94 |
| Compound 5 | 100 | 87 | 100 | 86 |
| Compound 6 | 102 | 103 | 100 | 100 |
| Compound 7 | 104 | 92 | 100 | 83 |

Table 2 demonstrated that test compounds 1 to 7 strongly inhibit CDK8 and CDK19.

Example 2: Enrichment of pancreatic progenitor cells at stage 4 with CDK8/19 inhibitor

1. Induction of differentiation of iPS cells into pancreatic progenitor cells

The human iPS cells used were Ff-WJ-18 cells (Kyoto University). The Ff-WJ-18 cells maintenance-cultured according to Reference Example 1 were induced to differentiate into pancreatic progenitor cells by the following procedures. Culture was performed at 37°C under 5% $CO_2$ in all cases, and the culture container used was coated with iMatrix-511(TM) (Nippi).

(1) Stage 1

Ff-WJ-18 cells (700,000 cells/9.6 $cm^2$) were seeded to RPMI1640 medium containing 1% penicillin/streptomycin, 1% B27, activin A (100 ng/mL), CHIR99021 (3 μM), and Y-27632 (10 μM), and cultured for 1 day. The cells were further cultured for 2 days in RPMI1640 medium containing 1% penicillin/streptomycin, 1% B27, and activin A (100 ng/mL) with the medium replaced with a fresh one every day.

(2) Stage 2

The resulting cells were cultured for 4 days using instead iMEM medium containing 1% penicillin/streptomycin, 1% B27, and KGF (50 ng/mL).

(3) Stage 3

The resulting cells were cultured for 2 days using instead iMEM medium containing 1% penicillin/streptomycin, 1% B27, KGF (50 ng/mL), noggin (100 ng/mL), KAAD-cyclopamine (cell-permeable analog of cyclopamine, hedgehog signal inhibitor (0.5 μM)), and TTNPB (arotinoid acid, RAR agonist (10 nM).

(4) Stage 4

The cells obtained at stage 3 were treated with 0.25% trypsin-EDTA and then dispersed by pipetting. After centrifugal separation of 0.25% trypsin-EDTA, the cells were suspended, then reseeded to iMEM medium containing (i) 1% penicillin/streptomycin, 1% B27, KGF (100 ng/mL), EGF (50 ng/mL), nicotinamide (10 mM), and Y-27632 (50 μM),

or (ii) iMEM medium containing 1% penicillin/streptomycin, 1% B27, KGF (100 ng/mL), EGF (50 ng/mL), and nicotinamide (10 mM), and cultured for 1 day. The cells were further cultured for 4 days in iMEM medium supplemented with 1% penicillin/streptomycin, 1% B27, KGF (100 ng/mL), EGF (50 ng/mL), nicotinamide (10 mM), and 0.00001 μM to 1 μM of each compound having CDK8/19-inhibiting activity described in Example 1. For comparison, similar experiments were conducted as to a medium supplemented with no compound having CDK8/19-inhibiting activity (negative control), and a medium supplemented with a CDK1/2 inhibitor (CAS443798-55-8 (217714, Merck), 0.00001 μM to 1 μM) instead of the compound having CDK8/19-inhibiting activity.

The cells after the culture at stage 4 were collected, and the proportion of NKX6.1-positive/PDX-1-positive cells was determined by flow cytometry. Likewise, the proportion of the cells was determined as to iPS cells and cells before the culture at stage 4 (s3d2: stage 3, day 2). The case of using (i) a medium containing a ROCK inhibitor (Y-27632 (50 μM)) at s3d0 (stage 3, day 0) is shown in Figure 2A (KENY50-KENT), and the case of using (ii) a medium containing no ROCK inhibitor (Y-27632) is shown in Figure 2B (KEN-KENT). As is evident from the results of Figure 2, all the compounds having CDK8/19-inhibiting activity basically exhibited a high proportion of NKX6.1-positive/PDX-1-positive cells in a dose-dependent manner as compared with the control.

Also, an effect was compared between a factor having CDK1/2-inhibiting activity and a factor having CDK8/19-inhibiting activity by a similar method. Specifically, a compound having CDK1/2-inhibiting activity (CAS443798-55-8 (217714, Merck)) or compound 1 having CDK8/19-inhibiting activity was added at 0.00001 μM to 1 μM to the cells obtained at stage 3, which were then cultured for 4 days. The proportion of NKX6.1-positive/PDX-1-positive cells was determined. The results are shown in Figure 3. As is evident from the results of Figure 3, efficient induction of differentiation into pancreatic progenitor cells was not observed when the compound having CDK1/2-inhibiting activity was allowed to act instead of the compound having CDK8/19-inhibiting activity.

A similar effect was observed when Ff-I01-S1 cells (Kyoto University) were used as human iPS cells, in addition to the Ff-WJ-18 cells. As seen from this, differentiation into NKX6.1-positive and PDX-1-positive pancreatic progenitor cells is efficiently attained by the addition of a factor having a CDK8/19-inhibiting effect, irrespective of the types of iPS cell lines.

Industrial Applicability

According to the present invention, the differentiation of pluripotent stem cells into NKX6.1-positive and PDX-1-positive pancreatic progenitor cells can be efficiently induced. The pancreatic progenitor cells obtained according to the present invention can be utilized as a cell medicine or device for treating a disease such as diabetes mellitus.

All publications, patents and patent applications cited herein are incorporated herein by reference in their entirety.

## Claims

1. A method for producing NKX6.1-positive cells, comprising culturing NKX6.1-negative cells in the presence of a factor having CDK8/19-inhibiting activity.

2. The method according to claim 1, wherein the NKX6.1-negative cells are cultured in the presence of the factor having CDK8/19-inhibiting activity and a growth factor.

3. The method according to claim 1 or 2, wherein the PDX-1-positive cells are enriched.

4. The method according to any one of claims 1 to 3, wherein the NKX6.1-positive cells are PDX-1-positive cells.

5. A cell culture comprising human cells and a factor having CDK8/19-inhibiting activity, wherein at least about 10% of the human cells are NKX6.1-positive and PDX-1-positive cells.

6. The cell culture according to claim 5, wherein at least about 50% of the human cells are NKX6.1-positive and PDX-1-positive cells.

7. The cell culture according to claim 5, wherein at least about 80% of the human cells are NKX6.1-positive and PDX-1-positive cells.

8. Human pluripotent stem cell-derived NKX6.1-positive and PDX-1-positive cells produced using a factor having CDK8/19-inhibiting activity.

**9.** A medicament comprising human pluripotent stem cell-derived NKX6.1-positive and PDX-1-positive cells produced using a factor having CDK8/19-inhibiting activity.

**10.** An inducer of differentiation into pancreatic progenitor cells, comprising a factor having CDK8/19-inhibiting activity.

**11.** A medium for induction of differentiation into pancreatic progenitor cells, comprising a factor having CDK8/19-inhibiting activity.

**12.** Use of a factor having CDK8/19-inhibiting activity as an inducer of differentiation into pancreatic progenitor cells.

# FIG. 1

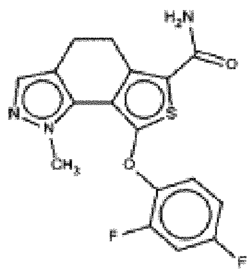

Compound 1

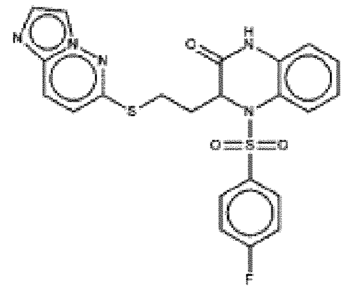

Compound 2

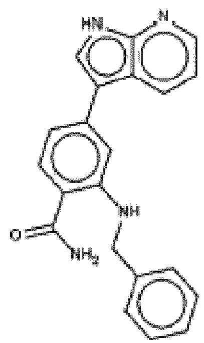

Compound 3

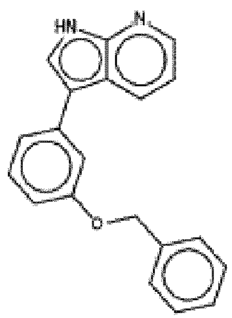

Compound 4

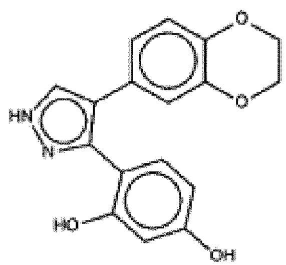

Compound 5

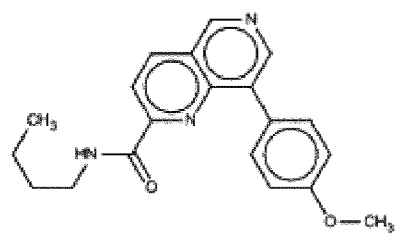

Compound 6

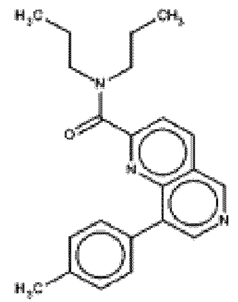

Compound 7

FIG. 2

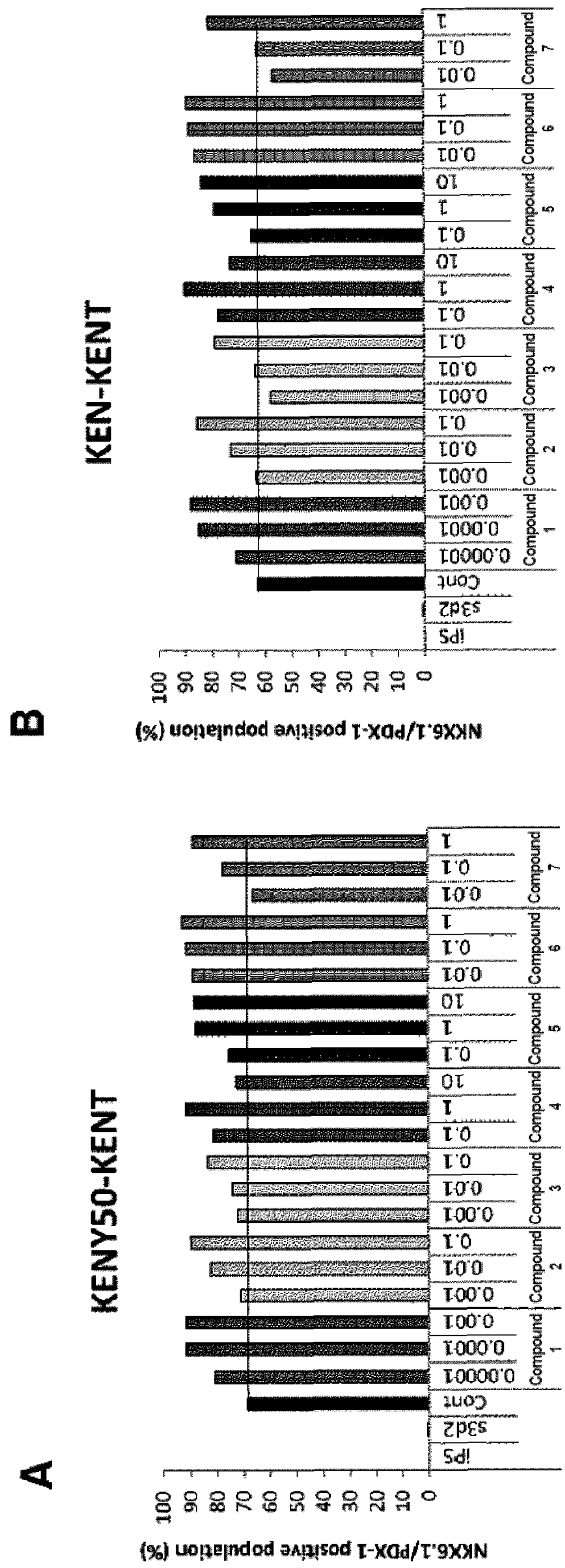
A
KENY50-KENT
B
KEN-KENT
NKX6.1/PDX-1 positive population (%)
0
10
20
30
40
50
60
70
80
90
100
iPS
s3d2
Cont
Compound 1
0.00001
0.0001
0.001
Compound 2
0.001
0.01
0.1
Compound 3
0.001
0.01
0.1
Compound 4
0.1
1
10
Compound 5
0.1
1
10
Compound 6
0.01
0.1
1
Compound 7
0.01
0.1
1

FIG. 3

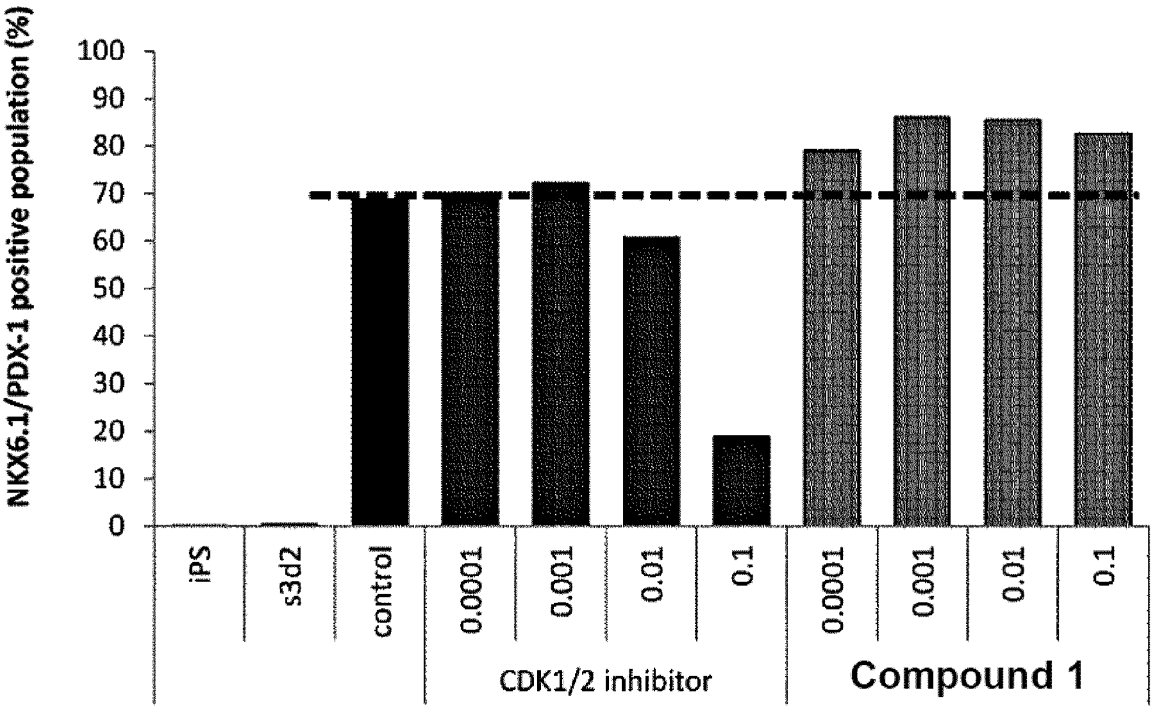
NKX6.1/PDX-1 positive population (%)
100
90
80
70
60
50
40
30
20
10
0
iPS
s3d2
control
0.0001
0.001
0.01
0.1
0.0001
0.001
0.01
0.1
CDK1/2 inhibitor
Compound 1

## INTERNATIONAL SEARCH REPORT

International application No. PCT/JP2018/007965

A. CLASSIFICATION OF SUBJECT MATTER
Int. Cl. C12N5/10(2006.01)i, A61K35/545(2015.01)i, A61P3/10(2006.01)i, C12N5/071(2010.01)i

According to International Patent Classification (IPC) or to both national classification and IPC

B. FIELDS SEARCHED
Minimum documentation searched (classification system followed by classification symbols)
Int. Cl. C12N5/10, A61K35/545, A61P3/10, C12N5/071

Documentation searched other than minimum documentation to the extent that such documents are included in the fields searched
Published examined utility model applications of Japan 1922-1996
Published unexamined utility model applications of Japan 1971-2018
Registered utility model specifications of Japan 1996-2018
Published registered utility model applications of Japan 1994-2018

Electronic data base consulted during the international search (name of data base and, where practicable, search terms used)
JSTPlus/JMEDPlus/JST7580 (JDreamIII), CAplus/MEDLINE/BIOSIS/WPIDS (STN), PubMed

C. DOCUMENTS CONSIDERED TO BE RELEVANT

| Category* | Citation of document, with indication, where appropriate, of the relevant passages | Relevant to claim No. |
|---|---|---|
| X<br>Y | WO 2015/178431 A1 (KYOTO UNIVERSITY) 26 November 2015, claims 1, 2, 13, 29, paragraph [0018] & US 2017/0175082 A1, claims 1, 2, 13, 29, paragraph [0048] & EP 3147353 A1 & CN 106536718 A | 1-12<br>1-12 |
| X<br>Y | JP 2012-507292 A (CENTOCOR ORTHO BIOTECH INC.) 29 March 2012, claim 1, paragraphs [0035], [0084], [0129], [0132], [0133] & US 2010/0112693 A1, claim 1, paragraphs [0045], [0097], [0142], [0145], [0146] & WO 2010/051223 A1 & EP 2346988 A1 & KR 10-2011-0077016 A & CN 102272291 A | 1-12<br>1-12 |

☒ Further documents are listed in the continuation of Box C.   ☐ See patent family annex.

* Special categories of cited documents:
"A" document defining the general state of the art which is not considered to be of particular relevance
"E" earlier application or patent but published on or after the international filing date
"L" document which may throw doubts on priority claim(s) or which is cited to establish the publication date of another citation or other special reason (as specified)
"O" document referring to an oral disclosure, use, exhibition or other means
"P" document published prior to the international filing date but later than the priority date claimed
"T" later document published after the international filing date or priority date and not in conflict with the application but cited to understand the principle or theory underlying the invention
"X" document of particular relevance; the claimed invention cannot be considered novel or cannot be considered to involve an inventive step when the document is taken alone
"Y" document of particular relevance; the claimed invention cannot be considered to involve an inventive step when the document is combined with one or more other such documents, such combination being obvious to a person skilled in the art
"&" document member of the same patent family

| | |
|---|---|
| Date of the actual completion of the international search<br>21.05.2018 | Date of mailing of the international search report<br>29.05.2018 |
| Name and mailing address of the ISA/<br>Japan Patent Office<br>3-4-3, Kasumigaseki, Chiyoda-ku,<br>Tokyo 100-8915, Japan | Authorized officer<br><br>Telephone No. |

Form PCT/ISA/210 (second sheet) (January 2015)

**INTERNATIONAL SEARCH REPORT**

International application No. PCT/JP2018/007965

C (Continuation). DOCUMENTS CONSIDERED TO BE RELEVANT

| Category* | Citation of document, with indication, where appropriate, of the relevant passages | Relevant to claim No. |
|---|---|---|
| Y | CEE V. J., et al., Cortistatin A is a High-Affinity Ligand of Protein Kinases ROCK, CDK8, and CDK11, Angew. Chem. Int. Ed., 2009, vol. 48, pp. 8952-8957, heading, table 1, Scheme 2, p. 8956, left column, paragraph 2 | 1-12 |
| Y | YINT et al., A Novel CDKg Inhibitor Shows Potent Antitumor Efficacy in Preclinical Hematologic Tumor Models, Mol. Cancer Ther., 2014, vol. 13, No. 6, pp. 1442-1456, fig. 1B | 1-12 |
| A | JP 2015-506376 A (SENEX BIOTECHNOLOGY INC.) 02 March 2015, entire text & US 2014/0038958 A1, whole document & WO 2013/116786 A1 & EP 2809324 A1 & CN 104363913 A & KR 10-2015-0023223 A | 1-12 |
| A | WO 2015/159938 A1 (TAKEDA CHEMICAL INDUSTRIES, LTD.) 22 October 2015, entire text & US 2017/0044132 A1, whole document & EP 3205645 A1 | 1-12 |
| A | JP 2016-503408 A (SELVITA SA) 04 February 2016, entire text & US 2015/0274726 A1, whole document & WO 2014/072435 A1 & EP 2917217 A1 & KR 10-2015-0091074 A & CN 104903321 A | 1-12 |
| A | WO 2016/009076 A1 (MERCK PATENT GMBH) 21 January 2016, entire text & JP 2017-522324 A & US 2016/0016951 A1 & EP 3169678 A1 & CN 106817900 A | 1-12 |
| A | JP 2016-504013 A (KYOTO UNIVERSITY) 12 February 2016, entire text & US 2016/0108366 A1, entire document & WO 2014/104403 A1 | 1-12 |
| P, X | WO 2017/047797 A1 (KYOTO UNIVERSITY) 23 March 2017, entire text (Family: none) | 1-12 |
| P, X | TOYODA T. et al., Rho-Associated Kinases and Non-muscle Myosin Its Inhibit the Differentiation of Human iPSCs to Pancreatic Endoderm, Stem Cell Reports, 08 August 2017, vol. 9, pp. 419-428, entire text | 1-12 |

Form PCT/ISA/210 (continuation of second sheet) (January 2015)

## REFERENCES CITED IN THE DESCRIPTION

*This list of references cited by the applicant is for the reader's convenience only. It does not form part of the European patent document. Even though great care has been taken in compiling the references, errors or omissions cannot be excluded and the EPO disclaims all liability in this regard.*

### Patent documents cited in the description


- JP 2017040219 A
- US 20080145889 A
- US 9234178 B
- US 20120071477 A
- WO 2015159937 A
- WO 2015159938 A
- WO 2013116786 A
- WO 20140038958 A
- WO 2014134169 A
- JP 2015506376 A
- US 20150274726 A
- US 20160000787 A
- WO 2016009076 A
- WO 20160016951 A
- WO 2016018511 A
- WO 2016100782 A
- WO 2016182904 A
- JP 2008307007 A
- JP 2008283972 A
- US 20082336610 A
- US 2009047263 A
- WO 2007069666 A
- WO 2008118220 A
- WO 2008124133 A
- WO 2008151058 A
- WO 2009006930 A
- WO 2009006997 A
- WO 2009007852 A
- WO 2001074823 A
- US 20050020591 A
- WO 2005095400 A
- WO 2014079787 A


### Non-patent literature cited in the description


- **TAKAHASHI K ; YAMANAKA S.** *Cell,* 2006, vol. 126, 663-676
- **TAKAHASHI K ; YAMANAKA S. et al.** *Cell,* 2007, vol. 131, 861-872
- **OKITA, K. ; ICHISAKA, T. ; YAMANAKA, S.** *Nature,* 2007, vol. 448, 313-317
- **NAKAGAWA M ; YAMANAKA S. et al.** *Nature Biotechnology,* 2008, vol. 26, 101-106
- **OKITA K et al.** *Nat. Methods,* May 2011, vol. 8 (5), 409-12
- **OKITA K et al.** *Stem Cells.,* vol. 31 (3), 458-66
- **YU J. ; THOMSON JA. et al.** *Science,* 2007, vol. 318, 1917-1920
- **PARK IH ; DALEY GQ.** *Nature,* 2007, vol. 451, 141-146
- **SHI Y. ; DING S. et al.** *Cell Stem Cell,* 2008, vol. 3 (5), 568-574
- **KIM JB. ; SCHOLER HR et al.** *Nature,* 2008, vol. 454, 646-650
- **HUANGFU D. ; MELTON, DA. et al.** *Nature Biotechnology,* 2008, vol. 26 (7), 795-797
- **TOYODA et al.** *Stem cell Research,* 2015, vol. 14, 185-197
- *Bioorganic & Medicinal Chemistry,* 2016, vol. 24 (11), 2466-2475